(19)

Europäisches
Patentamt
European
Patent Office
Office européen
des brevets

(11) **EP 3 988 659 A1**

(12) **EUROPEAN PATENT APPLICATION**

(43) Date of publication:
**27.04.2022  Bulletin 2022/17**

(51) International Patent Classification (IPC):
***C12N 15/63*** (2006.01)

(21) Application number: **20203857.6**

(22) Date of filing: **26.10.2020**

(52) Cooperative Patent Classification (CPC):
**C12N 15/1093; C12N 15/63; C12N 15/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Wageningen Universiteit
6708 PB Wageningen (NL)**

(72) Inventors:
- **NIEUWKOOP, Thijs
  6515 XS Nijmegen (NL)**
- **CREUTZBURG, Sjoerd Constantijn Arnoud
  6721 VW Bennekom (NL)**
- **VAN DER OOST, John
  6871 HZ Renkum (NL)**

(74) Representative: **V.O.
P.O. Box 87930
2508 DH Den Haag (NL)**

(54) **PROKARYOTIC POST STOP-CODON SEQUENCES**

(57)  The invention relates to methods of modulating gene expression in prokaryotes. The invention further relates to a prokaryotic expression cassette, a method of expressing a protein of interest in a prokaryotic cell, and to a prokaryotic cell comprising the prokaryotic expression cassette according to the invention.

EP 3 988 659 A1

**Description**

FIELD

The invention relates to methods of protein production in prokaryotes, and especially to methods for modulating production levels.

1 INTRODUCTION

Prokaryotes, including true bacteria or eubacteria, and archaeabacteria, often generate polycistronic mRNA for concerted transcription of functionally related genes, for instance for enzymes that compose metabolic pathways and for subunits of protein complexes (Galperin and Koonin, 2000. Nat Biotechnol 10:1038/76443; Huynen et al., 2000. Genome Res 10.1101/gr.10.8.1204). While transcription of the genes clustered in an operon is generally equal, differences in translation rate may cause differential expression of these genes at the protein level. Differential translation may arise from differences in ribosome binding efficiency, codon usage and impediments like strong secondary structures. Between successive genes in the operon, translational coupling has already been observed several decades ago (Oppenheim and Yanofsky, 1980. Genetics 95: 785-795; Schümperli et al., 1982. Cell 30: 865-871; Aksoy et al., 1984. J Bacteriol 157: 363-367). More recently, a systematic and quantitative characterisation of *E. coli* operons has shown that the expression of an upstream gene can influence the expression of a downstream gene, depending on the length of the intergenic region (Levin-Karp et al., 2013. ACS Synth Biol 2: 327-336).

Increased translation of an upstream gene by incorporating a range of ribosomal binding sites (RBS), each having different ribosome binding strength, has a direct or indirect effect on the translation rate of the downstream genes. Furthermore, it was found that translational coupling is also affected by so-called polar mutations in the upstream gene's coding sequence (Oppenheim and Yanofsky, 1980. Genetics 95: 785-795). For instance, a point mutation in the upstream gene *trpE* affects the expression of the downstream *trpD.*

Two main models have been proposed to explain this phenomenon. A first model is based on ribosomal "flow-through". Ribosomes terminating translation at the upstream gene's stop codon are in the direct vicinity of the downstream gene's initiation sites, where a direct feed-forward control may occur (Govantes et al., 1998. EMBO J 17: 2368-2377). This model was further confirmed by increasing the distance between the stop codon and the downstream start codon (Levin-Karp et al., 2013. ACS Synth Biol 2: 327-336). A decrease of translational coupling was found by increasing the length of the intergenic region. A second model is based on the helicase activity of 70S ribosome. Increased translation of an upstream gene can dissolve secondary structures throughout the operon, potentially removing inhibitory structures present on the downstream gene's initiation region. Strong secondary structures upstream of the *atpA* gene's translation initiation region were indeed found to inhibit the translation rate of atpA (Rex et al., 1994. J Biol Chem 269: 18118-18127). A model was proposed in which the secondary structure within the upstream *atpH* cistron is dissolved by the processive ribosome activity, also resulting in unfolding the downstream mRNA to improve accessibility for ribosomal binding to allow translation initiation of *atpA.* This translational feed-forward coupling phenomenon has important implications for designing operon synthesis as well as for operon reduction. The alterations of gene order and sequence not only affect translation rates of the altered gene but can also affect expression ratios throughout the operon, resulting in differential stoichiometries (Quax et al., 2013. Cell Rep 10.1016/j.celrep.2013.07.04924).

The efficiency of translation depends on many interconnected factors. Tuning of transcription, synthetically or in nature, occurs mainly through promoter sequence and the stabilization of the mRNA via the terminator sequence, which protects the mRNA from degradation by exoribonuclease. Translation efficiency is controlled by ribosome binding site (RBS) strength, mRNA stability, secondary structures and codon usage.

Tuning of expression in prokaryotes is required when building synthetic pathways within organisms. In addition, tuning of individual protein expression levels is desired to optimize many industrial biotechnological processes.

The promoter sequence can be changed to tune the transcription levels and consequently the amount of protein produced. However, for many non-model prokaryotes a limited number of promoter sequences is known.

The tuning of the ribosome binding site (RBS) is a commonly used way in biotechnology for tuning translation. However, the ribosome cannot bind the RBS if it is involved in a secondary structure. Therefore, tools are required that check and predict the effect of potential inhibitory structures. This makes the tuning of RBS for different genes less reliable. I.e. a "good" RBS for one protein is not necessarily a good RBS in combination with other protein sequences. A tool was developed for this purpose (Salis, 2011. Methods Enzymol 498: 19-42). While this tool was a great step forwards towards the prediction of expression based on the RBS strength, it is still far for perfect. This results in a trial-and-error approach when tuning or optimizing gene expression levels.

There is thus a need for tools tuning protein expression levels in prokaryotes.

2 BRIEF DESCRIPTION OF THE INVENTION

The 3' untranslated region (UTR), or post stop-codon, ante terminator region (proposed name: PSAT region), of a prokaryotic messenger RNA (mRNA) contains the nucleotide sequence in between an open reading frame (ORF) and a terminator sequence. The terminator halts transcription and stabilizes and protects the mRNA from degradation. Said terminator may act factor independent or factor dependent. Said factor independent terminator may be provided by an RNA structure that consists of a hairpin, normally a guanidine-cytidine (GC)-rich hairpin, which is followed by a stretch of uridine residues. In between the stop codon of the ORF, or in case of an operon the ORF located closest to the 3' end of the polycistronic transcript (mRNA), and the terminator resides a spacer sequence, the post stop-codon, ante terminator (PSAT) region or 3' UTR. The ribosome cannot reach the end of the protein sequence (stop codon) if a strong secondary structure of the terminator is too close to the stop codon. The secondary structure will sterically block the ribosome. Therefore, a spacer sequence, termed 3' UTR or PSAT, is often found at the 3' end of the mRNA of a gene.
It has now been found that this spacer sequence also has the potential to reliably tune the amount of protein that is being produced from a mRNA.
The invention therefore provides a method of modulating gene expression in prokaryotes, comprising (i) selecting a prokaryotic expression cassette expressing a protein of interest, said expression cassette comprising a promoter, optionally a 5' untranslated region (UTR) containing, for example, a ribosome binding site, a coding sequence for the protein of interest including a start codon and a stop codon, a 3' untranslated region (UTR) and a terminator sequence; (ii) replacing the nucleotide sequence of the 3' UTR between the stop codon of the protein of interest and terminator sequence with a randomized nucleotide sequence to generate a library of expression cassettes expressing the protein of interest; (iii) expressing the library of expression cassettes expressing the protein of interest in a prokaryote; and (iv) selecting an expression cassette with modulated expression of the protein of interest, preferably with increased or decreased expression of the protein of interest, when compared to the selected expression cassette under (i).
In an embodiment, said prokaryotic expression cassette expressing a protein of interest may comprise an operon expression more than one protein of interest.
By inserting a degenerate 30 nucleotide sequence in between the stop codon and the terminator sequence, a library of possible 3' UTR sequences may be generated. It was found that the expression of a reporter gene varied for different sequences. A range of sequences were extracted and said sequences were exchanged between reporters. Surprisingly, the effect of a specific 3' UTR sequence seemed independent of the reporter protein. A 3' UTR sequence that resulted in high expression in combination with one reporter protein also resulted in relative high expression for another reporter protein and *vice versa.* This effect was further verified by placing the sequences behind a third reporter protein, where after the same trends were observed.
This means that the 3' UTR can be used to tune translation reliably in a protein independent way. This may not be possible by tuning of the RBS sequence, because secondary structures between the RBS and the protein sequence may influence the initiation of translation, or the efficiency of translation. On the transcriptional level the promoter can be tuned. This is also a protein independent tuning method but, as mentioned earlier, in non-model organisms the amount of available promoter sequences may be limited for tuning gene expression levels by employing different promotor sequences.
Said randomized nucleotide sequence preferably is 20-100 nucleotides, more preferably about 25-50 nucleotides such as about 30 nucleotides. The length of the 3' UTR nucleotide sequence is based on a recent study that reported that a sequence smaller than 30 nucleotides can have a negative effect on the terminator's termination efficiency, while increasing the size above 30 nucleotides did not show any effect (Li et al., 2016. Nucleic. Acids Res. 44, 2554-2563). In addition, limiting the length of the 3' UTR may avoid the inclusion of unwanted sequences such as potential ribonuclease (RNAse) sites.
The invention further provides a method of modulating gene expression in prokaryotes, comprising (i) selecting an expression cassette expressing a protein of interest, preferably a prokaryotic expression cassette or a synthetic expression cassette for expressing a protein of interest; (ii) replacing the 3' untranslated region (UTR) between stop codon and terminator sequence for a nucleotide sequence depicted in Table 1; (iii) expressing the resulting expression cassettes to produce the protein of interest in a prokaryote; and (iv) selecting an expression cassette with modulated expression of the protein of interest, preferably with increased or decreased expression of the protein of interest, when compared to the selected expression cassette under (i).
The invention further provides a prokaryotic expression cassette, comprising an expression cassette for expression of a protein of interest, said expression cassette comprising a promoter, optionally a 5' UTR containing, for example, a ribosome binding site, optionally a poly-linker, a 3' untranslated region (UTR) comprising or consisting of a nucleotide sequence as depicted in Table 1, and a terminator.
Said terminator preferably is a Rho-independent terminator.
Said terminator preferably is or comprises the sequence 5'-CCCCGCTTCGGCGGGGTTTTTTT (SEQ ID NO.1).
The invention further provides a method of expressing a protein of interest in a prokaryotic cell, comprising

providing a prokaryotic expression cassette according to the invention; inserting a nucleotide sequence encoding a protein of interest in between the promoter and the 3' untranslated region (UTR) such that the promoter directs expression of the protein of interest in a suitable host prokaryotic cell; transforming a prokaryotic cell with the prokaryotic expression cassette comprising the nucleotide sequence encoding a protein of interest; and expressing the protein of interest.

Said nucleotide sequence encoding a protein of interest preferably is codon optimized or codon harmonized for expression in said prokaryotic cell. Said protein of interest may be a heterologous protein, which is normally not expressed in said prokaryotic cell, or a homologous protein which is normally expressed in said prokaryotic cell.

The invention further provides a prokaryotic cell, comprising the prokaryotic expression cassette according to the invention in which a nucleotide sequence encoding a protein of interest is inserted in between the promoter and the 3' untranslated region (UTR) such that the promoter directs expression of the protein of interest.

Said prokaryotic cell preferably is *Escherichia coli.*

Said nucleotide sequence encoding a protein of interest preferably is codon optimized for expression in said prokaryotic cell.

The invention further provides a method of expressing a protein of interest in a prokaryotic cell, comprising providing the prokaryotic cell comprising the prokaryotic expression cassette according to the invention; and expressing the protein of interest.

The invention further provides a method of producing a protein of interest in a prokaryotic cell, comprising providing the prokaryotic cell comprising the prokaryotic expression cassette according to the invention; and expressing the protein of interest thus producing the protein of interest.

Said method of producing a protein of interest in a prokaryotic cell preferably further comprises at least partly purifying the protein of interest.

In a preferred method of producing a protein of interest in a prokaryotic cell according to the invention, the protein of interest is tagged.

## 3 FIGURE LEGENDS

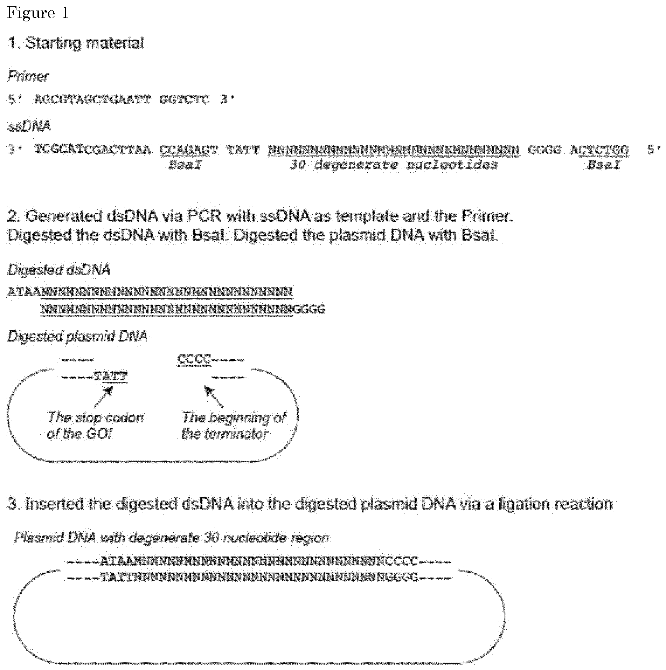

Figure 1. Schematic overview of randomized 3' UTR library generation.

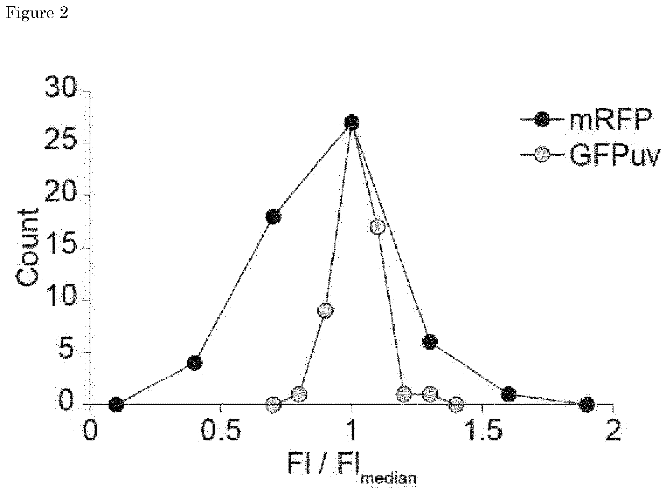

Figure 2. Histogram graph for the expression range of Green Fluorescent Protein (GFP) optimized for maximal fluorescence when excited by standard UV light (360-400 nm) (GFPuv) (Crameri et al., 1996. Nat Biotechnol 14, 315-9) (grey) and monomeric red fluorescent protein (mRFP) (Campbell et al., 2002. PNAS 99, 7877-7882) (black) of the 3' UTR region library. The spread of expression is shown as the Fluorescent Intensity (FI) divided by the Fluorescent Intensity median as determined separately for mRFP and GFPuv. Fluorescent intensity data points are also present in Table 2 (mRFP) and Table 3 (GFPuv).

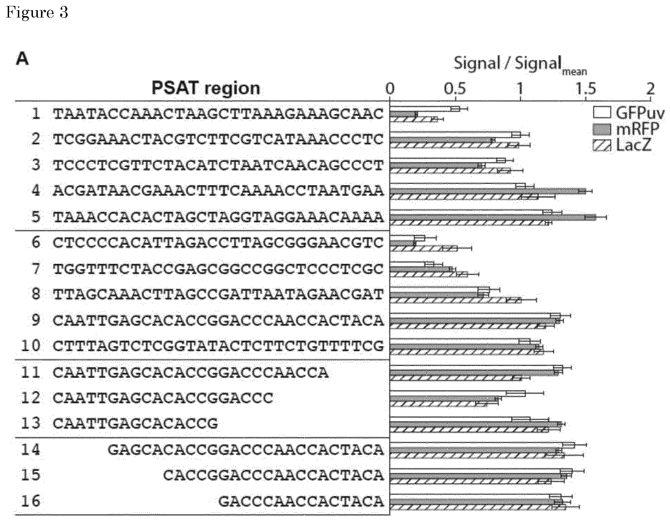

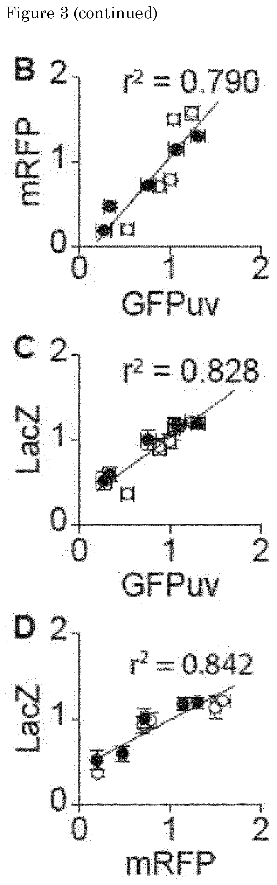

Figure 3. (A) Expression levels of GFPuv, mRFP and beta-galactosidase encoded by the *E. coli* LacZ gene with indicated post stop-codon, ante terminator (PSAT) 3' UTR sequences. Expression is shown as signal divided by the mean signal as determined for each of the markers separately. A level of fluorescence was was determined for GFPuv and mRFP, while the hydrolysis of o-nitrophenyl-β-D-galactopyranoside (ONPG) and extinction at 420 nm was used as a readout for beta-galactosidase. (B-D) Comparison of expression levels of (B) mRFP and GFPuv, (C) beta-galactosidase and GFPuv and (D) beta-galactosidase and mRFP with 3' UTR sequences SEQ ID NO.7, 14, 41, 58, 62 (white) and SEQ ID NO.63, 69, 117, 127, 134 (black). All data points showed a correlation, indicating that the effect the 3' UTR has on translation is mostly ORF independent.

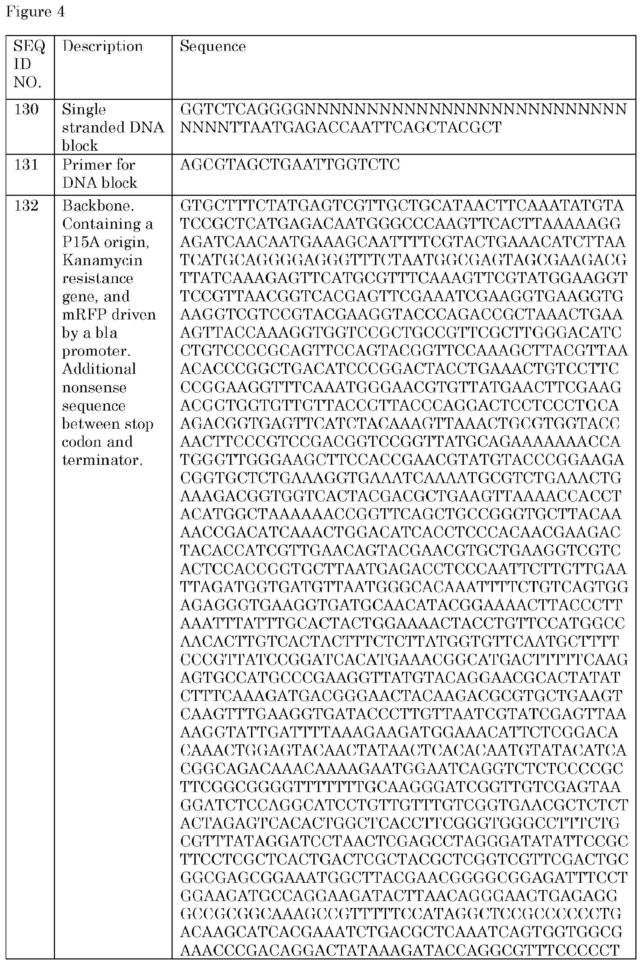

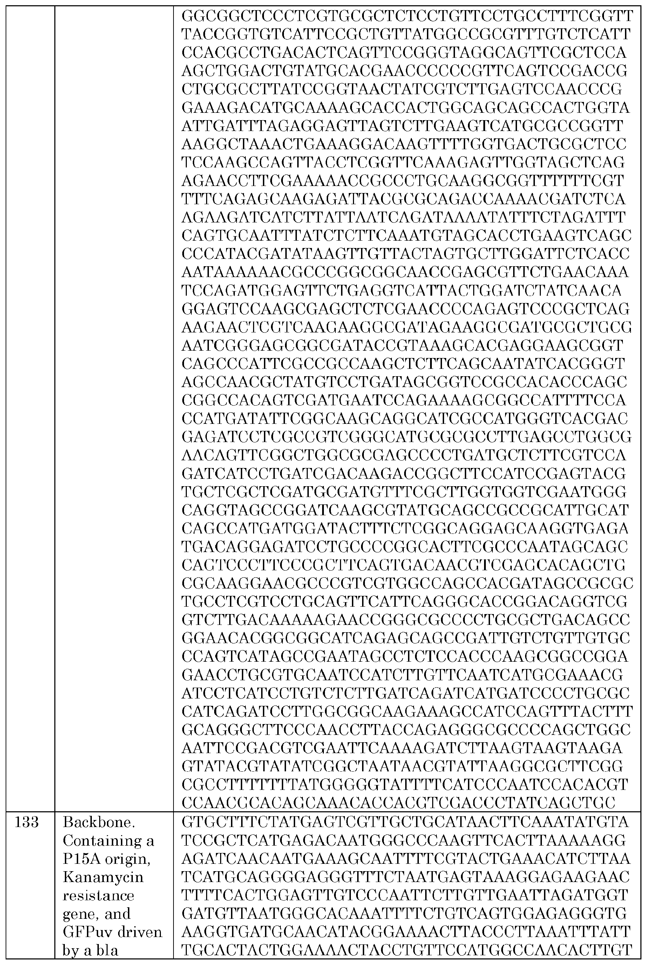

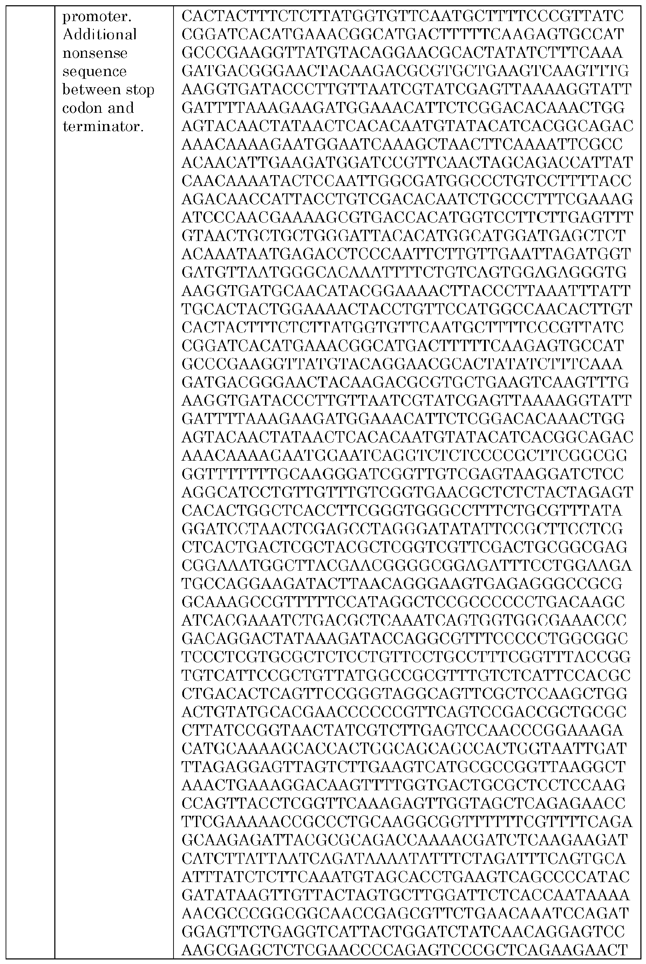

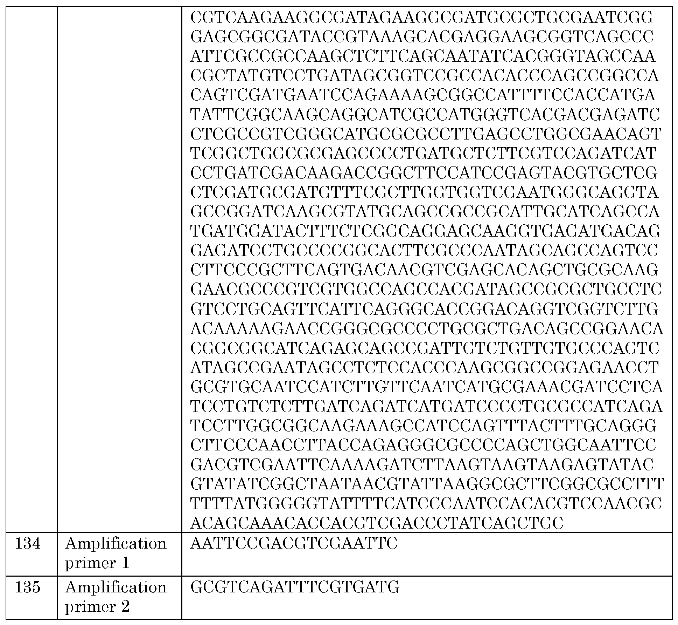

Figure 4. DNA sequences as used in Example 1.

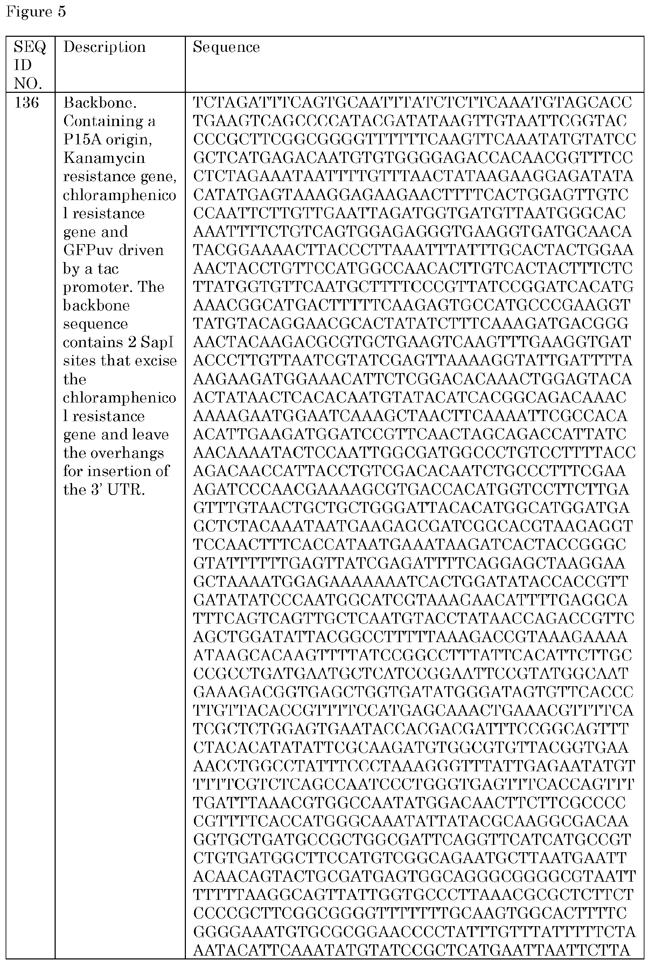

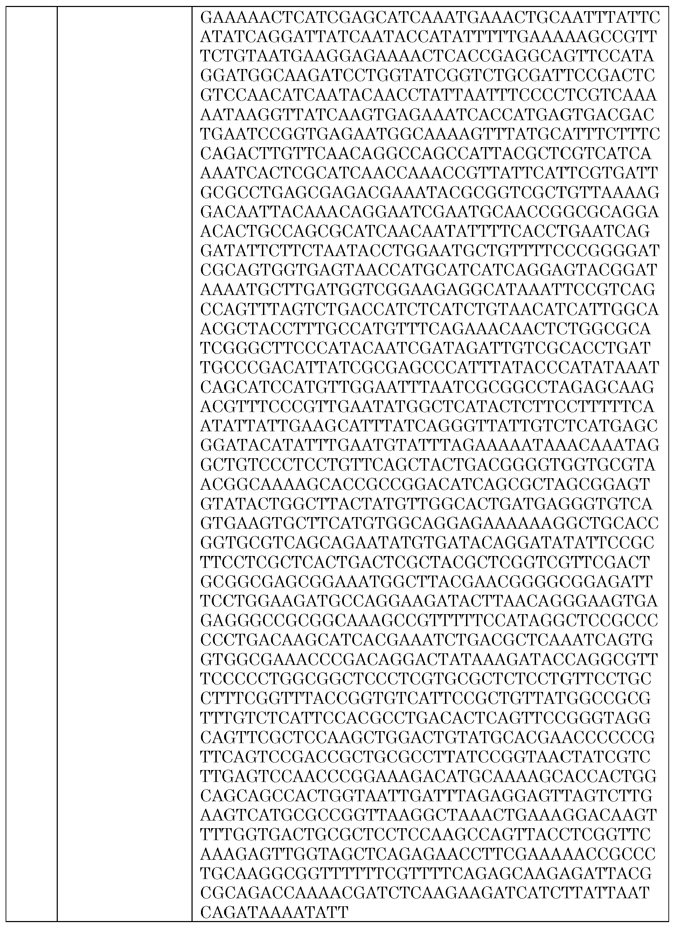

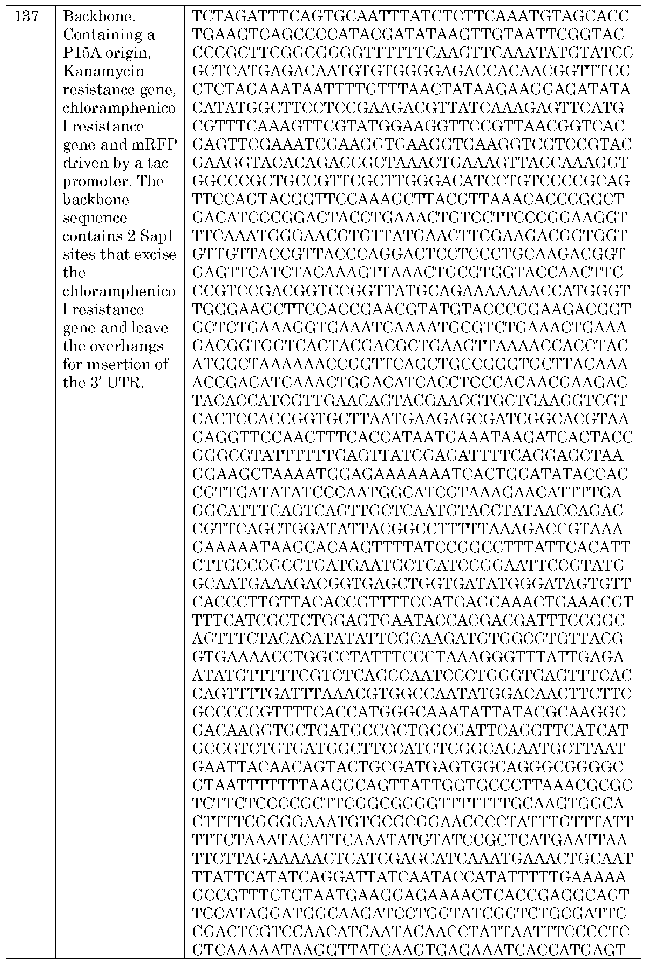

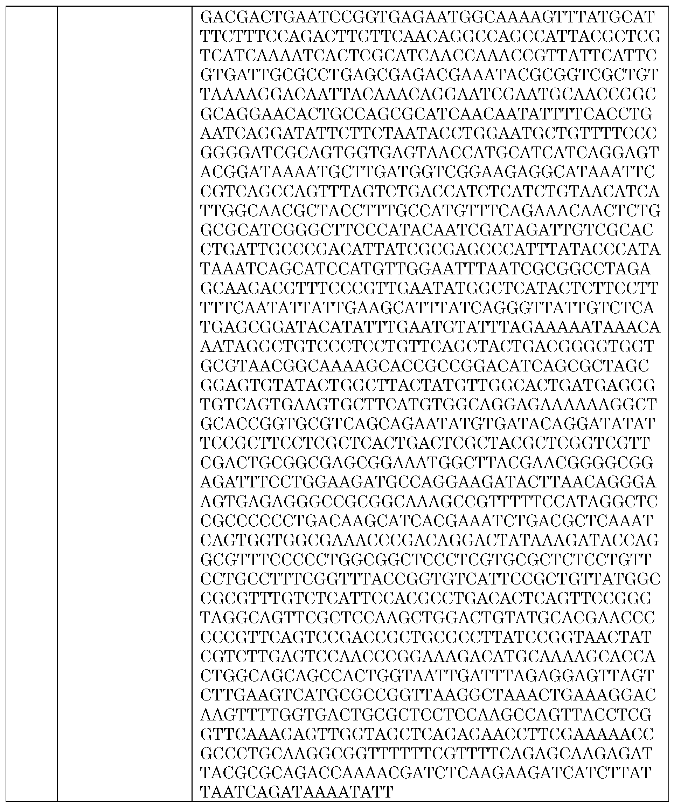

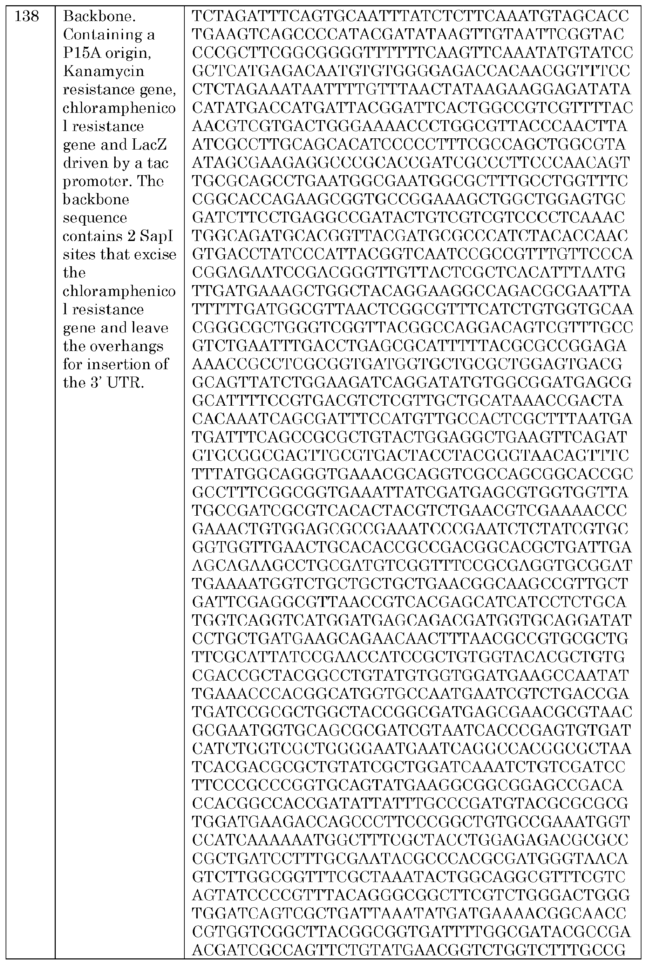

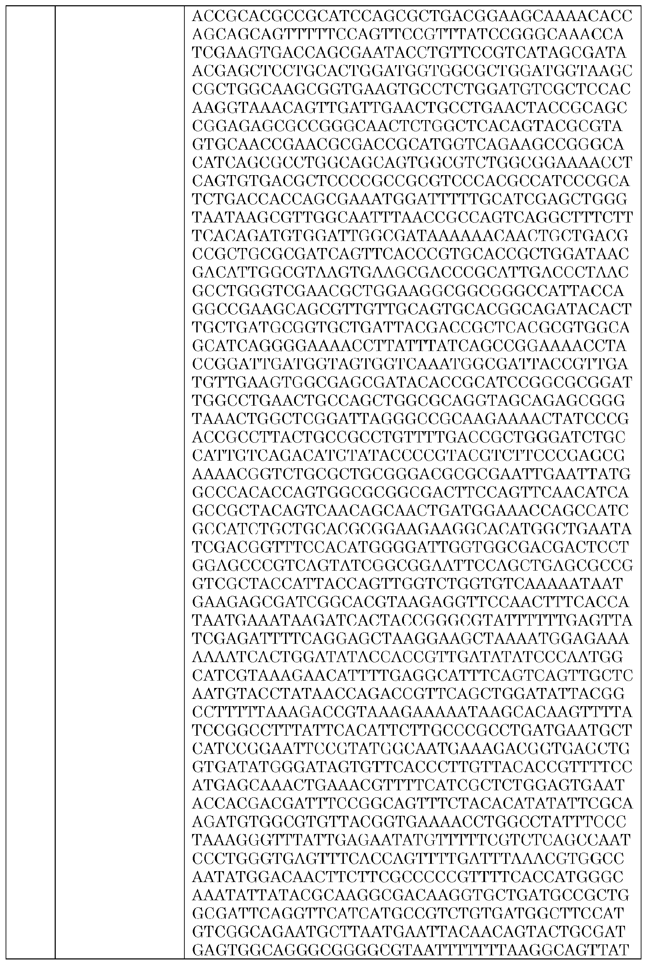

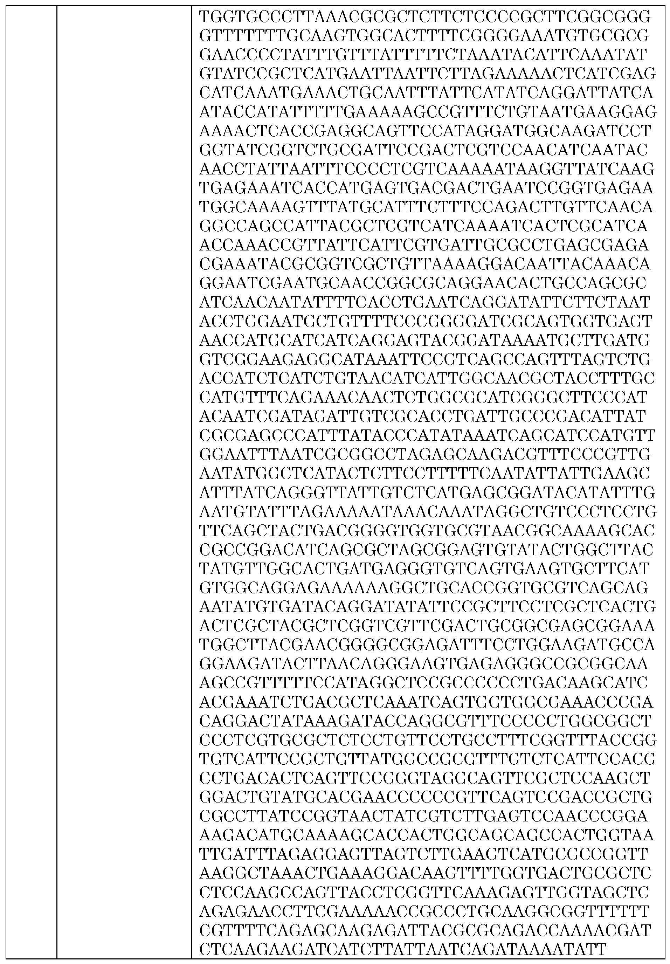

Figure 5. Vector sequences as used in Example 2. Marker genes are GFPuv, mRFP and LacZ.

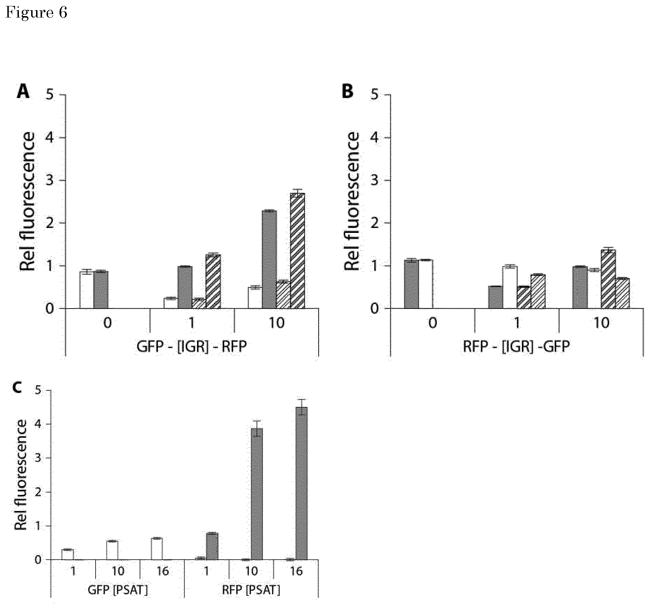

Figure 6. GFPuv and mRFP fluorescence in an operon with different intergenic regions (IGRs) between mRFP and GFPuv-expressing genes in one operon. The numbers on the X-axis (0, 1, 10, 16) indicate the PSAT region that was added to the IGR directly behind the stop codon. PSAT sequence numbers are indicated in Figure 3, whereby 0 is a control without any extra nucleotides. Solid: No terminator stem, and without poly U tract, behind the PSAT region. Dashed: With terminator stem, but without poly U tract, behind the PSAT region. White bars: GFPuv fluorescence. Grey bars: mRFP fluorescence. All fluorescence was normalised for the raw average of WT-GFP IGR-0. Error bars indicate the SD. (A) GFPuv followed by an IGR and mRFP. (B) mRFP followed by an IGR and GFPuv. (C) Controls with either GFPuv or mRFP.

4 DETAILED DESCRIPTION OF THE INVENTION

4.1 Definitions

The term "prokaryote" as is used herein, refers to a cellular organism that lacks an envelope-enclosed nucleus. Cellular organisms with an nucleus enclosed within a nuclear envelope are referred to as eukaryotes. Prokaryotes can be split up into two domains: *Bacteria* and *Archaea.* Bacteria comprise relatively simple genomes compared to eukaryotes. Most often, the bacterial genome consists of a single circular chromosome or a single linear chromosome, which makes bacteria ideal candidates for gene manipulation due to being a relatively simple vehicle. Preferably, *Escherichia coli* is used as prokaryote. *E. coli* is a Gram-negative, facultative anaerobic, rod-shaped, coliform bacterium of the genus *Escherichia* that is commonly found in the lower intestine of warm-blooded organisms (endotherms). Other prokaryotic organisms that can be used include Gram-positive and Gram-negative bacterial species such as species of the genus *Bacillus,* for example *Bacillus subtilis,* species of the *Clostridium* genus such as *C. acetobutilicum, C. autoethanogenum, C. beijerinckii, C. tyrobutyricum* and *C. saccharoperbutylacetonicum,* species of the genus *Streptomyces* such as *S. nodosus, S. hygroscopicus, S. avermitilis, S. verticillus, S. hygroscopicus, S. griseus, S. clavuligerus, S. venezuelae,,* and *S. viridochromogenes,* species of the genus *Staphylococcus,* for example *Staphylococcus aureus,* species of the genus *Corynebacterium,* for example *C. glutamicum,* species of the genus *Pseudomonas,* for example *Pseudomonas fluorescens, P. putida* and *P. auruginosa,* species of the *Amycolatopsis* genus such as *A. coloradensis, A. mediterranei,* and *A. orientalis,* and species of the genus *Lactococcus,* for example *Lactococcus lactis,*

Further suitable prokaryotic organisms include Archaea bacteria such as for example, extremophile archaea, which are resistant either to heat or to extremes of acidity and alkalinity, including *Pyrococcus* species such as *P. furiosus, Sulfolobus* species, *Thermofilum* species, *Thermococcus* species such as *T. coalescens, Thermoproteus* species, *Pyrobaculum* species, *Haloquadratum* species, *Thermoplasma* species and *Ferroplasma* species. A protein that is produce in an extremophile archaea tends to be very stable, for example in organic solvents, allowing their use in environmentally friendly processes in green chemistry.

Suitable prokaryotic organisms may be provided in Table 1 of Donohue et al., 2018 (Donohue et al., 2018. Trends Biotech 2: 134-146).

The term "expression cassette" as is used herein, refers to a genetic construct that enables expression of a gene of interest, or genes of interest, in a cell such as a prokaryotic cell. Said expression cassette comprises a promoter sequence, optionally a 5' UTR containing a ribosome binding site, a 3' untranslated region, a 'post stop-codon, ante terminator' (PSAT) region, and a terminator sequence. Said expression cassette preferably is present in a vector.

The term "vector" as is used herein, refers to a piece of DNA, either single or double stranded, such as a plasmid, a bacteriophage, or a phagemid (also termed phasmid). The vector can be for example, a plasmid, a viral genome or part thereof, or a combination thereof. The vector is used to transduce a gene encoding a protein of interest into a suitable prokaryotic host cell. Once in the host cell, the vector may replicate independently of, or coincidental with, the host chromosomal DNA. Alternatively, the vector can target insertion of at least an expression cassette comprising the transgene DNA into the host chromosome.

The term "3' untranslated region (UTR)", as is used herein, refers to the nucleotide sequence in between a stop codon of a gene of interest and a terminator. Said 3' UTR preferably has a certain length in order to allow ribosomes to reach the stop codon at the end of the protein sequence, and not to be hindered by a secondary structure of the terminator. Said 3' UTR may be 10-1000 nucleotides, preferably 20-100 nucleotides, preferably about 25-50 nucleotides such as about 30 nucleotides.

The term "5' untranslated region (UTR)", as is used herein, refers to the nucleotide sequence between transcription start and the initiation codon (start codon) of a gene of interest (or in case of an operon, the gene/ORF/cistron located closest to the 5' end of the polycistronic transcript (mRNA)). The 5' UTR is preferably from prokaryotic origin, and may typically be between 30 and 100 nucleotides long. The 5' UTR preferably comprises a Shine-Dalgarno (SD) sequence, which is a ribosomal binding site (RBS), generally located between 3 and 10 nucleotides upstream of the start codon, which normally is AUG. The RBS is responsible for the recruitment of a ribosome during the initiation of protein translation.

The term "coding sequence", as is used herein, refers to a portion of a gene's DNA or RNA that codes for a protein.

The term "terminator", as is used herein, refers to a sequence at the end of a gene that defines the end of a transcriptional unit. Prokaryotic termination mechanisms are either Rho-dependent or Rho-independent. Rho factor is a helicase which assists RNA polymerase in the termination of the transcript. A most commonly used terminator is a Rho-independent terminator. Rho-independent termination relies on the formation of a GC-rich hairpin in the RNA transcript followed by a single stranded poly-uracil tract. The tertiary structure of the hairpin-DNA complex is thought to destabilize the transcription complex, initiating release of a transcribing RNA polymerase. If required, multiple terminators may be used, such as double terminators, or triple terminators, to reduce unwanted transcription and translation of downstream elements.

The term "Rho-factor", as is used herein, includes reference to Rho-factor like proteins such as homologs and analogues of the Rho protein of *Escherichia coli.*

The term "gene", as is used herein, refers to a stretch of nucleic acid sequences that encode one or more expression products such as proteins. On a genomic level, the term gene includes reference to all nucleic acid elements that enable expression of the one or more expression products, including promoter/enhancer sequences, coding sequences, intron sequences if present, 5' and 3' untranslated sequences, and terminator sequences. On a transcript level, the term gene includes reference to nucleic acid elements such as coding sequences and 5' and 3' untranslated sequences. The term gene includes reference to an operon that encodes a polycistronic mRNA.

The term "promoter", as is used herein, refers to a nucleotide sequence at the 5' end of a gene onto which the transcription initiation machinery, including a RNA polymerase, binds and initiates transcription, Said promoter is often located 5-100 bp upstream of a start codon of gene. Promoters are often associated with regulatory elements, i.e. binding sites for potentially transcription activators and/or transcriptional repressors. Although promoters may vary among prokaryotic genomes, in eubacteria a -10 consensus sequence relative to the transcription start site (TSS), called the -10 (in *E. coli*: TATAAT) region and a -35 region (*in E. coli* TTGACA), are often present in prokaryotic promoters. The A-T-rich, -10 region is thought to facilitate unwinding of the DNA template. Archeabacteria often have a TATA box, or binding site for a TATA-like binding protein, at about -25 bases of a transcription start site, and a binding site for a Transcription Factor B (TFB) at about -30 to -35 of a transcription start site.

A promoter may be constitutively active, inducible, or repressible. In addition, the strength of a promoter may range from very low to very high.

A promoter sequence that can be used is the Amp (bla) promoter. It is a weak constitutive promoter for example used for expression of ampicillin resistance gene in *E. coli.* This promoter was originally found in transposon Tn2660 upstream of the initiation codon of the structural beta-lactamase (bla) gene (Chen and Clowes, 1984. Nucl Acids Res. 12, 3219-3234). Other commonly used *E. coli* promoters are the tac and trc promoters. Tac and trc are controllable promoters and are hybrid promoters derived from the lac and trp promoters (Brosius, 1984. Gene 27, 151-160.). The tac promoter consists of the -35 region of the trp promoter and the -10 region of the lac promoter. The trc promoter was created by insertion of 1 base pair into the 16 base pair sequence between the consensus -10 and -35 sequences of the tac promoter, in order to obtain an optimal consensus distance of 17 base pairs between the -35 and -10 sequences. They are stronger promoters than either lac and trp because their sequences are more like the consensus sequence. Just as with lac, both the trc and tac promoters are inducible by lactose and isopropyl-$\beta$-D-thiogalactoside (IPTG). Vectors that use one of these promoters may also carry the lacO operator and the lacI gene, which encodes the repressor. Other commonly used *E. coli* promoter sequences are the bacterial phage T3, T7 and SP6 promoters, which are to be used in combination with the respective, host-encoded, phage RNA polymerases, either as such or in combination with regulatory elements including for instance a lac operator, a promoter of the arabinose operon, a promoter of the rhamnose operon, a promoter of the tryptophan operon, a promoter of the lac operon, a bacteriophage lambda promoter, as is known to a person skilled in the art.

Promoters for other prokaryotes are also well known, such as the $P_{veg}$, $P_{aprE}$, $P_{amyE}$, and $P_{P43}$ promoter for *B. subtilis* (Kim et al., 2008. Biotechnol Bioprocess Engineering 13: 313). For *Lactobacillus,* examples of promoters include the *slpA* promoter and the *orfX* promoter (Sørvig et al., 2005. Microbiol 151: 2439-2449). Algorithms are available that may help to predict promoter regions in other prokaryotes (de Avila e Silva et al., 2011. J Theor Biol 287: 92-99; de Jong et al., 2012. BMC Genomics 13: 299). As is known to the person skilled in the art, promoters of known genes can be used in genetic manipulation (Guiziou S. et al., 2016. Nucl Acids Res 44: 7495-7508) to generate altered promoters such as stronger promoters, or promoters that also function in other prokaryotes.

The term "gene expression", as is used herein, refers to the process by which information from a gene is used in the synthesis of a functional gene product. Said gene product is often a protein, and sometimes a functional RNA, such as transfer RNA (tRNA), ribosomal RNA (rRNA) or small anti-sense RNA (ssRNA). The process of gene expression comprises multiple steps and can be modulated at many levels. The mechanism of gene expression comprises transcription, translation, and possible post-translational modification.

The term "modulating", as is used herein in the context of gene expression, refers to regulating or adjusting a level of gene expression to a certain measure or proportion. Modulating expression of a single gene, either increasing or decreasing expression level of a gene, may enhance the overall yield of a product, especially of a functional protein. Tuning of expression of individual genes is often required when building synthetic pathways within organisms, such as for industrial biotechnological processes. Fine tuning of individual expression levels of proteins may enhance the overall yield of a product. The term modulating refers to increasing or decreasing expression of a protein of interest.

The term "poly-linker", as is used herein, refers to short segment of DNA which contains many restriction enzyme recognition sequences. A poly-linker is also known as a multiple cloning site (MCS). The purpose of a poly-linker in a vector is to facilitate a piece of DNA to be inserted into that region without disrupting the rest of the vector. A poly-linker is present in a variety of vectors, for example in pUC vectors, e.g. pUC18 and pUC19, pSF-pA-PromMCS or pSF-OXB20-BetaGal (Sigma-Aldrich). In expression vectors, a poly-linker is often located downstream of a promoter sequence to allow insertion of a nucleotide sequence encoding a protein of interest. Expression of the protein of interest in a suitable prokaryotic host cell is preferably controlled by the promoter sequence.

The term "transduce" and variants thereof such as "transduction", as is used herein, refers to insertion of exogenous genetic material into a prokaryotic cell. The term transducing includes the transformation of nucleic acid molecules by a nucleic acid molecule such as a plasmid. The taking up of genetic material, preferably of nucleic acid molecules such as DNA or RNA, is either by natural or artificial means. Artificial means include the generation of competent cells to render them passively permeable to genetic material, for example by incubation in the presence of calcium chloride and the application of a heat shock, and electroporation by which cells are shocked with an electric field of preferably 10-20 kV/cm.

The term "codon optimized", as is used herein, refers to the selection of codons in a sequence encoding a protein of interest for optimal expression of the protein of interest in a prokaryotic host cell. A single amino acid may be encoded by more than one codon. For example, arginine and leucine are each encoded by a total of 6 codons. Codon optimization, i.e. the selection of a preferred codon for a specific organism at every amino acid residue, plays a critical role, especially when proteins are expressed in a heterologous system. While codon optimization may play a role in achieving high gene expression levels, other factors such as secondary structure of the messenger RNA also need to be considered. Codon optimization is offered by commercial institutions, such as ThermoFisher Scientific, called Invitrogen GeneArt Gene Synthesis, GenScript, called GenSmart™ Codon Optimization, or GENEWIZ, called GENEWIZ's codon optimization tool.

The term "codon harmonized", as is used herein, refers to the alignment of codon usage frequencies with those of the expression host particularly within putative inter-domain segments where slower rates of translation may play a role in protein folding. Codon harmonization may be accomplished by algorithms such as provided by Angovet al., 2011 (Angovet al., 2011. In: Evans and Xu (eds) Heterologous Gene Expression in E.coli. Methods in Molecular Biology (Methods and Protocols), vol 705. Humana Press; Claassens et al., 2017. PLoS One 12: e0184355.

The term "operon", as is used herein, refers to two or more genes that are transcribed together into one mRNA strand under control of one promoter. Several genes may be co-transcribed to define an operon. An operon provides a complete package for gene expression and synthesis of proteins. Said proteins within an operon are often related, in that they are required for a specific function and/or are synthesized in response to a specific stimulus. Examples of operons in *Escherichia coli* include the Lac operon which is involved in lactose degradation, and the Trp operon which is involved in tryptophan biosynthesis. An operons may be under negative control, positive control, or both. For example, an operon may be blocked by a repressor molecule in the absence of an inducer. When the inducer is present it interacts with the repressor protein, releasing it from the operator and allowing transcription to proceed.

The term "purifying the protein", as is used herein, refers to the purification of one or more proteins, which comprises a series of processes intended to isolate one or more proteins from a complex mixture, usually cells, tissues, and/or growth medium. Various purification strategies can be followed. For example, proteins can be separated based on size, for example in a method called size exclusion chromatography. Alternatively, proteins can be purified based on charge, e.g. through ion exchange chromatography or free-flow-electrophoresis, or based on hydrophobicity (hydrophobic interaction chromatography). It is also possible to separate proteins based on molecular conformation, for example by affinity chromatography. Said purification may involve the use of a specific tag, for example at the N-terminus and/or C-terminus of the protein. After purification, the proteins may be concentrated. This can for example be carried out with lyophilization or ultrafiltration.

The term "tag" or "tagged", as is used herein, refers to the addition of an oligo- or polypeptide, for example to the amino (N) or carboxy (C) terminus of a protein. The addition of a tag allows to isolate or immobilize a protein. Commonly used tags include a poly-histidine tag such as a 6x(His) tag, a myc tag, a glutathione-S-transferase tag, a HiBiT tag (Promega, Madison, Wisconsin), a FLAG tag, a HA tag, or multimeric tags such as a triple FLAG tag (WO2001027293). A proteinase recognition sequence may be positioned in between the protein of interest and a tag allowing removal of the tag when required.

Definitions of common terms in cell biology and molecular biology, as are used herein, can be found in The Encyclopedia of Molecular Biology, published by Blackwell Science Ltd., (1994) (ISBN 0-632-02182-9); Benjamin Lewin, (2009) Genes X, published by Jones & Bartlett Publishing, (ISBN-10: 0763766321); Kendrew et al. (eds.) (1995), Molecular Biology and Biotechnology: a Comprehensive Desk Reference, published by VCH Publishers, Inc., (ISBN 1-56081-569-8) and Coligan et al., eds., 2009. Current Protocols in Protein Sciences Wiley Intersciences.

Unless otherwise stated, the present disclosure can be performed using standard procedures, as described, for example in Sambrook et al., (2014) Molecular Cloning: A Laboratory Manual (4 ed.), Cold Spring Harbor Laboratory Press, Cold Spring Harbor, N.Y., USA; Davis et al., (1995) Basic Methods in Molecular Biology, Elsevier Science Publishing, Inc., New York, USA; and Berger and Kimmel Eds, (1987). Methods in Enzymology: Guide to Molecular Cloning Techniques Vol. 152, S. L., Academic Press Inc., San Diego, USA, which are all incorporated by reference herein in their entireties.

4.2 Methods for screening a random 3' UTR library

4.2.1 Generating a random 3' UTR library

A random 3' UTR library may be generated by providing a single stranded DNA (ssDNA) containing at least 15 degenerate nucleotides, such as 15-100 degenerate nucleotides, preferably 20-50 degenerate nucleotides, more preferably about 30 degenerate nucleotides. Said random 3' UTR library may, for example, be generated by chemical synthesis using, for example, the phosphoramidite method of Marvin H. Caruthers (Caruthers, 1985. Science 230: 281-285).

A random 3' UTR library may be provided as a single stranded nucleotide library, which is subsequently converted into a double stranded DNA library. As an alternative, or in addition, said random 3' UTR library may be generated by as a single stranded nucleotide library by employing enzymatic synthesis using a terminal deoxynucleotidyl transferase, followed by conversion into a double stranded DNA library, or directly as a double stranded DNA library by random mutagenesis employing, for example, a low fidelity DNA polymerase.

Said degenerate nucleotides may be flanked on both sides with recognition sites for one or more restriction enzymes. Restriction enzymes can be either Type I, II, III, IV or V enzymes, or artificial restriction enzymes (Enghiad and Zhao, 2017. ACS Synth Biol 6: 752-757). Preferably, Type II enzymes are used. Most preferably, Type IIS enzymes are used. Examples of restriction enzymes and their recognition sites include BsaI, BsmBI, BbsI, EcoRI, EcoRII, BamHI, HindIII, TaqI, NotI, HinF1, Sau3AI, PvuII, SmaI, HaeIII, HgaI, AluI, EcoRV, EcoP15I, KpnI, PstI, SacI, SalI, ScaI, SpeI, SphI, StuI, XbaI, SapI. Other restriction enzymes can be found in databases such as REBASE (New England Biolabs), as is commonly known in the art. A nucleotide spacer flanking the degenerate nucleotides on one or both sites of the 3' UTR library may be provided to create a sequence or sequences that will be restricted by the chosen restriction enzyme, while the degenerate nucleotides are not cut, or only rarely cut, when said chosen restriction enzyme is applied. For this, a restriction enzyme with seven or even eight base pair recognition sequences such as SapI or CspCI (both available from New England Biolabs) may preferably be used. Additionally, a fixed region of, for example, 10-30 nucleotides, such as 15-25 nucleotides, preferably 17-21 nucleotides, may be provided at one or both ends of the ssDNA, to allow primer annealing in order to generate a double stranded randomized 3' UTR library. Said fixed region may overlap with the nucleotide spacer flanking the degenerate nucleotides on one or both sites of the 3' UTR library, as will be clear to a person skilled in the art.

Said ssDNA may be converted into a double stranded DNA (dsDNA) nucleotide sequence using, for example, amplification methods such as polymerase chain reaction (PCR) or isothermal amplification. In case of PCR, an adequate primer or pair of primers may be provided, which may be complementary to at least part of the fixed region of the ssDNA. The molar ssDNA:primer ratio may be 0.1-2, preferably 0.25-1, more preferably about 0.5. PCR is a widespread method for exponentially amplifying DNA sequences. This is performed by thermal cycling by denaturing the DNA strain into two single-stranded DNA molecules at an elevated temperature (94-98 °C). When the temperature is lowered to about 50-65 °C, a primer or pair of primers are able to bind to the single-stranded DNA, resulting of annealing of the primers to the single-stranded DNA. The primer or primers are chosen specifically for the DNA sequence that is to be multiplied. In a third part of the thermal cycle, the complementary part of the single strand DNA is generated by elongating the primer, by providing the mixture with a DNA-dependent DNA polymerase, for example *Thermus aquaticus* (Taq) polymerase, and free deoxyribonucleotides (dNTPs). This last step is carried out at a temperature suitable for the DNA polymerase. The cycle is started anew after this third step. Preferably at least 5 cycles of amplification are performed, more preferably at 10-30 amplification cycles. such as about 20 cycles.

As an alternative, said ssDNA may be converted into a double stranded DNA (dsDNA) nucleotide sequence using, for example, a primer and a polymerase, without a denaturation step. In this way, the ssDNA may be converted into double stranded DNA without amplification of the resulting dsDNA. Amplification may result in a bias towards specific sequences and the resultant library may be a true randomized library. To further reduce or prevent amplification of the 3" UTR dsDNA) nucleotide sequences, only one single primer may be employed for the step of converting the ssDNA into double stranded DNA.

Preferably at least cycles of annealing an elongating are performed, without a denaturation step, more preferably at least 40 cycles such as at least 70 cycles up to a total of about 99 cycles.

The resulting dsDNA may be purified from other components, such as excess primers, nucleotides, DNA polymerase, and salts. Purification of dsDNA may, for example, be carried out with a spin column containing a silica matrix to which DNA can be selectively bound in the presence of chaotropic salts. Other reaction components, such as enzymes, nucleotides, detergents, and primers either will not bind well or are removed during subsequent washing steps. Finally, the DNA may be eluted from the column under low-salt conditions. Preferably, the purification of dsDNA is carried out with a commercial kit such as, for example, provided by Sigma-Aldrich, ThermoFisher Scientific or New England BioLabs. Other methods for the purification of oligonucleotides include high-performance liquid chromatography, polyacrylamide gel electrophoresis and affinity chromatography. Alternative purification methods including organic extraction, such as phenol chloroform extraction, followed by ethanol precipitation, or PEG purification, may be used as well.

Isothermal amplification of DNA has the advantage over PCR that a constant temperature can be used. Examples of isothermal amplification methods include helicase-dependent amplification (HDA) (Vincent et al., 2004. EMBO Rep 5: 795-800), loop-mediated amplification (LAMP) (Notomi et al., 2000. Nucleic Acids Res 28: E63), nucleic acid sequences-based amplification (NASBA) (Guatelli et al., 1990. Proc Natl Acad Sci USA 87: 1874-1878), rolling circle amplification (RCA) (Ali et al., 2014. Chem Soc Rev 43: 3324-3341), strand-displacement amplification (SDA) (Walker et al., 1992. Nucleic Acids Res 20: 1691-6) and recombinase polymerase amplification (RPA) (Piepenburg et al., 2006. PLoS Biology 4: e204)

A double stranded, random 3' UTR library may be inserted into a suitable vector. For this, a vector may be provided comprising a gene coding for a protein of interest. Said protein of interest preferably is a protein which can be detected, preferably electrochemically, colorimetricly and/or luminescently, including fluorescently. Examples of such detectable proteins include green fluorescent protein (GFP) and its derivatives, such as enhanced GFP (eGFP), recombinant GFP (rGFP), red fluorescent protein (RFP), and yellow fluorescent protein (YFP), β-galactosidase, alkaline phosphatase, β-glucuronidase activity, and luciferase. Other fluorescent protein variations include GFPuv, a Green Fluorescent Protein optimized for maximal fluorescence when excited by standard UV light (360-400 nm) (Crameri et al., 1996. Nat. Biotechnol 14, 315-9) and mRFP, a monomeric red fluorescent protein (mRFP) (Campbell et al., 2002PNAS 99: 7877-7882).

As an alternative, or in addition, said detectable protein is an antibiotic resistance marker such as kanamycin resistance marker, ampicillin resistance marker etc. Transduced cells may be grown on higher than normal, such as extremely high, antibiotic levels, allowing growth of only those transduced bacteria that have increased expression, i.e. higher than average expression levels, of the antibiotic resistance marker.

Said vector may be chosen from a plasmid, a bacteriophage, a phagemid or phasmid. More preferably, the vector is a plasmid. Plasmids include p15A, ColE1, pBR322, RSF1030 and CloDF13. Further suitable vectors are provided, for example, under the headers "Protein purification" and "Controlled expression" at addgene.org/collections/bacterial-expression/. Said vector may further comprise at least one origin of replication and, optionally, a selection marker such as an antibiotic resistance gene or a sensitizer such as thymidine kinase, which renders the host cell sensitive to ganciclovir selection. Possible antibiotic resistance genes include resistance genes against penicillin or derivatives thereof, aminoglycoside, chloramphenicol, macrolides such as erythromycin, azithromycin and clarithromycin, streptothricin, tetracycline, quinolone, rifampin, sulfonamide, trimethoprim, kanamycin, phleomycin D1 such as zeocin, and streptomycin. Another selection marker type for positive selection is reversal of auxotrophy, for example by cloning *his*B+ in a *his*B auxotroph prokaryotic cell and growing it in a medium lacking histidine.

The vector further comprises a promoter for expression of the protein of interest in a suitable prokaryotic host cell. Said promoter may be a constitutive promoter or an inducible promoter, and may provide low, medium or high expression levels of a gene of interest.

The vector further optionally comprises an additional dummy sequence between the stop codon of the gene encoding the protein of interest, preferably a detectable marker, and the terminator sequence. Said dummy sequence preferably does not code for a specific product and has preferably a substantial length, for example 50-800 nucleotides, preferably 300-600 nucleotides, such as about 450 nucleotides. Said dummy sequence may be flanked by outward facing recognition sites for a specific restriction enzyme between the stop codon and terminator. Said dummy sequence may help to improved cloning efficiency of the double stranded, random 3' UTR library into a vector of choice.

The vector may be digested with a restriction enzyme for which a recognition site is present in both the vector flanking the PSAT sequence, optionally the dummy sequence, and flanking the sequences of the double stranded, random 3' UTR library. Said vector can then be purified and concentrated. Said purification may be performed as described above, for example through a gel, preferably an agarose gel. The dummy sequence may thus be separated from the rest of the vector , as the rest of the vector is longer than said dummy sequence and can thus easily be separated from the dummy sequence.

The double stranded, random 3' UTR library may also be digested with the same restriction enzyme. The digested dsDNA may be inserted into the digested vector via a ligation reaction, for example employing a T4 DNA ligase as is known to a person skilled in the art.

As an alternative to classical restriction enzyme ligation (e.g. Roche) cloning methods, said insertion can be performed with techniques known to the person skilled in the art, for example with Golden Gate assembly (New England BioLabs), Gateway Cloning (Thermo Fisher).

A molar ratio of insert to vector is preferably between 5:1 and 1:10, more preferably 1:1-1:3, most preferably about 1:2. After insertion the vector may again be purified, according to methods as mentioned above, if needed.

4.2.2 Cell transduction with random 3' UTR library

The resulting vector may be introduced into prokaryotic cells. As is known to a person skilled in the art, cell competency is required for cells to take up extracellular DNA such as a plasmid by transformation. Many prokaryotic cells are naturally competent, such as *Bacillus subtilis, Lactobacillus lactis,* and *Staphylococcus aureus* (Lorenz and Wackernagel 1994. Microbiol Rev 58: 563-602). Other prokaryotes, most notably *E. coli,* may not be naturally competent and may be rendered competent through various methods. Said competent cells may for example be purchased from manufacturers such as ThermoFisher Scientific, Sigma Aldrich or New England BioLabs, or non-competent cells may be made competent with techniques such as calcium chloride treatment. Protocols are known to the person skilled in the art, and can for example be found on at international.neb.com/protocols/0001/01/01/electroporation-protocol-c2986 or at mybiosource.com/learn/testing-procedures/cacl2-transformation-technique/. Possible competent cells that are commercially available include DH10B, DH5$\alpha$, and BL21(DE3) (*E. coli),* and all prokaryotes that are natural competent, such as *Bacillus subtilis, Lactobacillus lactis,* and *Staphylococcus aureus.*

As an alternative, or in addition, introduction of a vector encoding a protein of interest, preferably a detectable marker, and comprising a random 3' UTR library, may be introduced into prokaryotic cells by, for example, electroporation (Wirth et al., 1989. Mol Gen Genetics 216: 175-7), biolistic particle delivery (gene gun), or infection in case of the vector is a bacteriophage.

Following introduction of a vector encoding a protein of interest, preferably a detectable marker, and comprising a random 3' UTR library, into prokaryotic cells, said cells may optionally be grown in an appropriate culture medium, such as lysogeny broth (LB), bile esculin or mannitol salt. A suitable medium for growth of the prokaryotic cells are commercially available, for example from ThermoFisher Scientific, New England BioLabs and Sigma Aldrich. A person skilled in the art is able to find an ideal medium in resources such as Collins and Lyne's Microbiological Methods: Eight Edition, published by Arnold (2004) (ISBN 0 340 80896 9). Said medium preferably comprises the antibiotic for which an antibiotic resistance gene may be included in the vector, or another marker which was included in the vector such as a selection marker, e.g. *hisB*+ in a *hisB* auxotroph prokaryotic cell, as is known by the skilled person in the art. This allows for selection of cells in which the resulting vector was successfully introduced. Said cells are incubated at an appropriate temperature, for example 37 °C, for an appropriate duration, e.g. 6-24hrs, preferably overnight.

Said medium preferably is a solid medium that is, for example, solidified by addition of gelatine or agar-agar. Following introduction of a vector, the resulting prokaryotic cells are preferably plated in a suitable solid medium to allow single cells to grow into colonies that can be picked for further analysis, as is known to a person skilled in the art.

Said transformed cells may be selected based on the chosen detectable marker, such as fluorescence intensity for a fluorescent protein marker. For each marker multiple cells may be picked and grown and incubated again in a cell medium, optionally comprising the selection marker such as an antibiotic. Conditions may be similar to the conditions in the previous step or, optionally, another or an additional selection marker may be chosen. Preferably, more than 50 individual cells or colonies for each marker are chosen. More preferably, more than 100 cells are chosen for each marker. Most preferably, a multitude of 96 cells is chosen, such as 96, 192,288 or 384 cells are chosen for each marker to determine a level of expression of the detectable marker in said cell, in relation to the sequence of the 3' UTR.

In a next step, individual cells are grown and the expression level of the chosen marker may be measured, preferably of at least 100 single progeny cells, preferably of at least 10.000 single progeny cells. For example, if said chosen marker is a fluorescent protein, the fluorescent intensity at least 10.000 single progeny cells, more preferred of at least 25.000 single progeny cells may be determined. The fluorescence can for example be measured with a flow cytometer equipped with the appropriate excitation and emission filters, depending on the fluorescent spectrum of the fluorescent protein. Alternatively, a plate reader may be used to determine the fluorescence of a whole culture. The determined level of expression of the individual progeny cells may then be normalized, for example by subtracting background fluorescence, and an average level of expression may be determined for each individual 3' UTR sequence. Said level of expression may be provided as relative level of expression, for example as $\leq 0.01$ x average, 0.1 x average, 1 x average, 10 x average and $\geq 100$ x average for each selectable marker.

Based on the resulting expression levels of the selectable marker in individual cells, cells may be selected to cover the whole range of expression levels of the marker. Said selection can comprise at least 2 individual cells, preferably at least 5 individual cells, more preferably at least 10 individual cells, such that a wide range of expression levels of the marker is presented by the individual cells. Said selected cells may be reinoculated and grown and incubated again under the same conditions as specified above.

The expression level of the marker may then be measured again for each of the selected cultures and the DNA region containing the promoter, 5' UTR, Open Reading Frame (ORF) and 3' UTR can be isolated. This isolation can be performed by colony amplification such as colony polymerase chain reaction (PCR), preferably according to the manufacturer's protocol or according to standard protocol such as, for example, provided by Sigma Aldrich, New England BioLabs, or GenScript. Primers can be selected that are compatible with the used vector, as is known by the person skilled in the art and can for example be found on at dnaseq.co.uk/resources/primers/compatibility-with-common-plasmids or on primer-BLAST (NCBI). Preferably, vector DNA may be released from the cells by heating the mixture to 90-100°C, more preferably 95°C for 8-12 minutes, more preferably 10 minutes, prior to an amplification reaction such as a PCR reaction. PCR may involve primer annealing at a temperature of 60-64 °C, more preferably 62°C, an elongation time of 80-120 seconds, more preferably 100 seconds at 70-72°C, and denaturing at 90-95°C. It is preferred that at least 20 PCR cycles, more preferably at least 30 PCR cycles, most preferably 35 PCR cycles are performed.

Said resulting amplified 3' UTR DNA may be sequenced. The totality of individual sequences that enable a wide range of expression levels of a marker provides a 3' UTR sequence sub-library. Potential sequencing methods include traditional methods such as Sanger sequencing (Sanger and Coulson, 1975. J Mol Biol 94: 441-8) and Maxam-Gilbert sequencing (Maxam and Gilbert, 1977. PNAS USA 74: 560-64), High-Throughput Sequencing (HTS) techniques such as Ion Torrent sequencing and Illumina sequencing, or Third Generation Sequencing (NGS) techniques such as pyrosequencing (Nyren and Uhlen, 1993. Anal Biochem 208: 171-175), single molecule real time (SMRT) sequencing (Levene et al., 2003. Science 299: 682-6) and nanopore DNA sequencing (Pennisi, 2012. Science 336, 534-537).

4.2.3 Validation of the 3' UTR sub-library

Said sub-library may subsequently be validated in order to verify that the identified 3' UTR sequences function independently from, for example, the detectable marker that was used to provide a certain level of expression. Validation may be carried out by generating phosphorylated oligonucleotides of the identified PSAT sequences and its associated complementary part. Suitable overhangs on either side of the double stranded phosphorylated oligonucleotides may be provided to allow easy cloning in one or more further vector.

A vector may be provided, comprising an origin of replication, a promoter, a protein of interest, preferably at least one selection marker such as an antibiotics resistance gene, and at least one recognition site for a restriction enzyme, in such a way that the resulting overhang ends are complementary to the overhang of the selected 3' UTR oligonucleotides. Said protein of interest preferably is a detectable protein, for example selected from green fluorescent protein (GFP) and its derivatives, such as enhanced GFP (eGFP), recombinant GFP (rGFP), red fluorescent protein (RFP), and yellow fluorescent protein (YFP), β-galactosidase, alkaline phosphatase, β-glucuronidase activity, and luciferase. Said protein of interest preferably differs from the detectable protein that was used for screening of the complete 3' UTR library as is indicated herein above under 4.2.2, or includes the detectable protein that was used for screening under 4.2.2, and at least one, preferably at least two, further detectable proteins.

Individual sequences of the 3' UTR sub-library may be introduced into the vector by any method known in the art, for example by classical restriction enzyme ligation (e.g. Roche), Golden Gate assembly (New England BioLabs), or Gateway Cloning (Thermo Fisher). The resulting vectors are subsequently introduced into prokaryotic cells, and individual cells are grown and incubated.

Optionally, each of the constructs may be pre-cultured in order to ensure equal growth of each culture. Pre-culturing may preferably be carried out in the presence of the compound for which a marker is present on the plasmid, such as an antibiotic in case of an antibiotics resistance gene. Said pre-culture is incubated overnight, preferably covered with a gas-permeable membrane. Incubation is preferably performed at 37°C, for preferably 6-24 hours in an appropriate cell medium which is known to the person skilled in the art. Said cell medium is preferably specific for the prokaryotic cells that are used, to ensure optimal growing conditions.

After the optional pre-culturing, the cells may be cultured, optionally under the same conditions.

After culturing the prokaryotic cells, the expression levels of the protein or proteins of interest are to be determined. Expression levels of individual cells expressing a protein of interest and comprising the same 3' UTR sequence, may be compared to identify 3' UTR sequences that function independently from the protein of interest in providing a certain level of expression. It will be clear to a person skilled in the art that said expression levels preferably refer to relative expression levels, that are normalized for the specific protein of interest.

4.3 Exchanging the 3' UTR

As is indicated in example 2, PSAT sequences have been isolated that modulate an expression level of a protein, independent from the nature of the protein.

A protein of interest may be expressed internally in a prokaryotic cell and isolated from a cell, secreted into the growth medium and isolated therefrom, or both. By placing a signal sequence in front of the coding sequence of a protein of interest, the expressed protein may be directed to a particular export pathway. Said export pathway may result in accumulation of the protein of interest in the periplasmic space, in the growth medium, or both, depending on the nature of the protein. Known export pathways in *E. coli* include the SecB-dependent, the twin-arginine translocation, and the signal recognition particle pathway (Green and Mecsas, 2016. In: Kudva et al. (eds) "Bacterial Secretion Systems: An Overview", ASM Science, pp 213-239).

The invention therefore relates to the regulation of protein expression in prokaryotes by exchange of the PSAT sequence for a PSAT sequence from a 3' UTR sub-library, which has been shown to modulate protein expression to a desirable protein expression level.

At least 3, preferably at least 5 DNA sequences may be selected from each 3' UTR sub-library. Said selection may be made in such a way to cover a full or near complete range of expression levels of said proteins of interest. For said selected DNA sequences, phosphorylated oligonucleotides of the PSAT sequence and its associated complementary part may be generated. Said sequences are preferably generated by a solid-phase chemical synthesis using, for example, the phosphoramidite method (Caruthers, 1985. Science 230: 281-285). Overhangs on either side of the double stranded phosphorylated oligonucleotides may be provided to enable introduction of a selected 3' UTR sequence in a suitable vector.

A vector may be provided, comprising an origin of replication and an expression cassette comprising promoter, a 5' UTR sequence, a gene encoding a protein of interest, and a terminator sequence. Said vector preferably comprises at least one marker such as an antibiotic resistance gene, and at least one recognition site for a restriction enzyme, in such a way that the resulting overhang ends are complementary to the overhang of the oligonucleotides.

Said protein of interest can be any protein that one may want to produce in a prokaryote. Of particular interest might be commercially interesting proteins, such as enzymes *sdhABC* and *acn* involved in the production of glutamate and lysine (Brinkrolf et al., 2010. J. Biotech 149: 173-82), cytokines such as interleukins, chemokines and growth factors (Cytokine and Protein User's Guide Book, R&D systems (2018)), alcohol acyltransferase such as Atf1, AtfA and Eat1, esterases; such as Eat1, Eht1 and Eeb1, custom recombinant protein production (Ferrer-Miralles, & Villaverde, 2013. Microb Cell Fact 12: 113) or other commercially relevant enzymes such as a lipase, a protease, an amylase, a phytase, and/or a cellulase (Baltaci et al., 2017. Geomicrobiol J 34: 53-62). In addition, protein complexes, such as for example CRISP/Cas ribonuclear effector complexes (Staals et al., 2013. Mol. Cell 52: 135-145; Staals et al., 2014. Mol. Cell 56: 518-530) may be efficiently produced at the required relative amounts in a prokaryotic cell using the methods of the invention. For each of said proteins, a 3' UTR sequence can be selected that modulates the expression of the protein to a requested level of expression.

Said prokaryotic expression cassette expressing a protein of interest may comprise an operon expressing more than one protein of interest. A specific 3' UTR sequence may be provided after the stop codon of a gene at the 3' border of the operon to modulate expression of all genes within that operon. In addition, specific 3' UTR sequences may be provided after the stop codon of each gene within an operon. As is shown in the examples, expression levels of individual genes within an operon, as well as the overall levels of expression of all genes within an operon, can be modulated by altering individual 3' UTR sequences after one or more stop codons of individual genes, including the 3' PSAT region.

It is noted that increasing the expression of a protein of interest in a prokaryotic cell does not necessarily increase a level of expression of a functional protein of interest. For example, overexpression of a protein may result in improper folding of that protein. Overexpression of a protein in *E. coli* often results in the presence of that protein in so called inclusion bodies, which proteins are to be refolded *in vitro* to become functional. A reduced level of expression for a specific protein may result in increased expression of a properly folded, active protein.

Alternatively, said protein of interest may be part of a prokaryotic metabolic pathway for which accurate tuning of the expression level between different proteins may be desirable. Such pathways include, for example, the production of a biofuel such as ethanol, 1-butanol and isopropanol, for which enzymes such as acetyl-CoA acyltransferase (thl), acetoacetyl-CoA transferase (ctfAB), and acetoacetate decarboxylase (adc) play an important role (Peralta-Yahya and Keasling, 2010. Biotech J 5: 147-162), the breakdown of cellulose, which includes the tuning of expression levels of, for example, endoglucanases and cellulase (Sadhu and Tushar, 2013. Cellulase production by bacteria: a review.Microbiol Res J Int 3: 235-258), and production of biopolyesters, such as polyhydroxyalkanoates (PHA), for which expression of, for example, arginine dihydrolase and β-galactosidase needs to be carefully tuned (de Paula et al., 2017. J. of King Saud Univ-Science 29: 166-173).

An individual sequence of the 3' UTR sub-library, which directs expression of a protein of interest at a desired level, may be introduced into the vector by any method known in the art, for example by classical restriction enzyme ligation (e.g. Roche), Golden Gate assembly (New England BioLabs), or Gateway Cloning (Thermo Fisher). The resulting vectors are subsequently introduced into prokaryotic cells, and individual cells are grown and incubated.

After culturing the transformed cells, the expression levels of the protein of interest may again be determined.

5 EXAMPLES

Example 1: random 3' UTR generation

Methods and materials

If not indicated otherwise, all chemicals were obtained from Sigma-Aldrich (Merck KGaA, Darmstadt, Germany).

A schematic representation of the random 3' UTR generation is provided in Fig. 1. The sequences used, and their sequence identification numbers, are presented in Figure 4. Single stranded DNA (ssDNA) containing 30 degenerate nucleotides, flanked on both sides with a 4 nucleotide extension (overhang) followed by a BsaI recognition sequence (SEQ ID NO.130) was obtained from Integrated DNA Technologies (Coralville, Iowa) and converted to a double stranded DNA (dsDNA) sequence using PCR and a primer (SEQ ID NO.131) that binds to a region adjacent to the randomized sequence. 200 pmol ssDNA and 400 pmol primer was used in a 50 μl reaction (NEB M0482). 99 cycles of primer annealing and elongation, without a denaturation step, were performed according to the standard temperatures as described by NEB and an annealing temperature of 57°C and elongation phase of 5 seconds. The resulting dsDNA sequences were purified and concentrated using a silica column to a final volume of 15 μl mQ (Zymo D4004). A vector, containing red fluorescent protein as a marker (SEQ ID NO.132) or green fluorescent protein as a marker (SEQ ID NO.133), was prepared by first inserting a substantial piece (450nt) of nonsense (dummy) DNA flanked by outward facing BsaI sites between the stop codon and terminator. The vector was digested with BsaI-HF®v2 (NEB R3733) which resulted in two fragments of 2991 and 468 nucleotides. The digested vector (2991 nucleotides) was purified and concentrated from a 1% agarose gel to a final volume of 10 μl (Zymo D4002). The dsDNA 3' UTR sequences were inserted into the vector using a NEB® Golden Gate Assembly Kit (NEB E1601) with a 3:1 ratio of insert to vector at 37°C for 1 hour. The Golden Gate mixture was purified and concentrated using a silica column to a final volume of 6 μl in deionized water (Zymo D4004). 1 μl was transformed by electroporation into DH10B cells (ThermoFisher Scientific C640003) according to standard protocol (ThermoFisher Scientific). The transformation mixture was plated on LB agar (lOg NaCl, 10g Peptone, 5g Yeast extract, 15g Agar, per 1L of distilled $H_2O$ ($dH_2O$)) plates containing 50 μg/mL Kanamycin and was incubated at 37°C overnight. For both the mRFP and GFPuv library 288 colonies each were selected based on visual fluorescent expression and each colony was individually grown in 200 μl LB (lOg NaCl, 10g Peptone, 5g Yeast extract, per 1L $dH_2O$) (50 μg/mL kanamycin) in 2 mL 96 well plates. The plates were incubated at 37 °C overnight on a shaker at 1000 rpm (1.5mm stroke). For each culture 50.000 single cells were measured. The fluorescence was quantified using a Attune NxT Flow Cytometer (561 nm excitation / 620±15 nm emission filter for mRFP, 405 nm excitation / 512±25 nm emission filter for GFPuv) (ThermoFisher Scientific, USA). Based on the fluorescent data of all 288 colonies a selection was made of 96 colonies that covered the full fluorescent range that was measured. These colonies were reinoculated and regrown under the same conditions. The selected 96 colonies for each fluorescent protein were measured again and the DNA region containing the promoter, 5' UTR, Open Reading Frame (ORF) and 3' UTR was extracted using colony polymerase chain reaction (PCR). 1 μl culture was used in a 25 μl Q5 polymerase (NEB M0492) reaction with two primers (SEQ ID NO 134 and 135) according to standard protocol (NEB). Plasmid DNA was released from the cells by heating the mixture to 95°C for 10 minutes prior to the PCR reaction. Primer annealing temperature was 62°C, an elongation time of 100 seconds was used, and 35 PCR cycles were performed. The PCR mixture containing the amplified DNA fragment was send for clean-up and sequencing by Macrogen Europe (Netherlands). Primer (SEQ ID NO.135) was used as a sequencing primer to obtain the 3' UTR sequence.

Results

Only sequence data of good quality, indicated as a Phred score of greater than 20 (Ewing et al., 1998. Genome Res 8: 175-185) and where the 3' UTR contained exactly 30 nucleotides was used. For mRFP this resulted in 56 sequences (out of 96), for GFPuv in 72 sequences (out of 96). Plotting the relative protein expression level of mRFP and GFPuv for each of these sequences, a range of different expression levels was observed (Figure 2). The raw sequence and expression data is provided in Table 1 (mRFP) and Table 2 (GFPuv), wherein the 3' UTR sequences are sorted from high to low expression level. It thus follows that expression level of these proteins depends on the 3' UTR sequence, and not on the length of said sequence, since all sequences were of equal length.

Table 1. Raw expression levels and sequence results for mRFP.

| SEQ ID NO. | Expression level (mRFP) | 3' UTR sequence |
|---|---|---|
| 2 | 98994 | TAAACCACACTAGCTAGGTAGGAAACAAAA |
| 3 | 90809 | GCGAAGTCCAACACTCCACCAAGAATCTAC |
| 4 | 89434 | TCAAACACAATTCAATCTACAGCAAACAAG |
| 5 | 85051 | AAGAGAGCAGTAGAGGCGTCAGCAGGACAC |
| 6 | 84717 | TAACCAAGCAAAGAAACCACATCCCACT AA |
| 7 | 82093 | TAACGACAATCACGGTGTGAGAAATCTATC |
| 8 | 81512 | TCTGGCATTCCTCCCGGCGTGCTACCTCAT |
| 9 | 77448 | ACGATAACGAAACTTTCAAAACCTAATGAA |
| 10 | 76806 | TAAAACCGCGAAAGCATCACAACAAACCAA |
| 11 | 75679 | GGAGGTAAAGATAGTCAAACACAACAAGAA |

(continued)

| SEQ ID NO. | Expression level (mRFP) | 3' UTR sequence |
|---|---|---|
| 12 | 74712 | ACAAAACTCAGAGGAAAAGAAGAAAACAAA |
| 13 | 74281 | GGAAACCGCAGACGATAAATAGGAGCAAAA |
| 14 | 73571 | AGGACGTTCGAAGGTAACAATATGAGAAAT |
| 15 | 72939 | AGTGGCAGCTCAGCATCCTTTGTACCCTAA |
| 16 | 72748 | AATGGCACAACATCCAAAATCTAAAACCAC |
| 17 | 72500 | CCGACACGAATGGCCGACCGAAGTAATACA |
| 18 | 71527 | TCCTAAGATCACCTTTCCATCCTAACCGAC |
| 19 | 70909 | TTACCAAAATCCAACTCAACAAAGAAATAT |
| 20 | 70063 | AACACATTATCTCACTTTTAATCACGTTAA |
| 21 | 70046 | AAAACCGCACAAATATCCAATAGGCGCAAA |
| 22 | 70022 | ATAAGAAATAAAACAAAGTAAGTAAGATCA |
| 23 | 69908 | TCGCACGGCTCAATGTGCAAATTACACCCA |
| 24 | 69856 | CAAATGTTCGCGCGCAGTGCGCGTTGGTAG |
| 25 | 69460 | GCACATATGAGCAGTACGAGAGCAATATAA |
| 26 | 69409 | CAAAACAAAGGACACGCCAAAATAATTTAC |
| 27 | 68868 | TAACTCTCAAGACGCGATACCAAAACATAA |
| 28 | 68078 | ATAACCTGACATACCCCTAAGATAACCGTG |
| 29 | 68042 | GACACCTCCCAAACCACTGCACCTTGAACC |
| 30 | 68006 | AAGCAATACACAGATAATAAACACACAAAT |
| 31 | 67633 | AAGAAATAAACCATAACCAAAATCAGTGTG |
| 32 | 64287 | ACGGCTCTTTGAGACGTCATGTTATACTCC |
| 33 | 63889 | TGGGCCTGGAGCGCCACCGTACCGGAGGAG |
| 34 | 63853 | CCGTTATGATATCCCTCTTAAACATTCTAC |
| 35 | 62205 | AACATGGCTACGGATCCAATGCCACATGTT |
| 36 | 57534 | TCCCTCGTTCTACATCTAATCAACAGCCCT |
| 37 | 54974 | ACATGCTGAGTTTTCGAATCGGATCGAAAA |
| 38 | 52651 | TACCCTTCTTCAGCTGCTTTCCAACCCTCC |
| 39 | 50340 | ACAGAATGCGTGGGCGCGGAAGGAGCAAGC |
| 40 | 50039 | CCATGAATCGTCTGCGTCGCTGGGGCTCTC |
| 41 | 49081 | CCTCTCCTATATTCCTCCACCGCATGCTAT |
| 42 | 49015 | TAATGAGGTCACGGTGTGCTGGAAGGGTGT |
| 43 | 48558 | ACGAACCTTTCCACTCCCCAATTCTTAAGT |
| 44 | 48462 | ATGAAAACTACAAAATTCATCAACTAAACT |
| 45 | 47859 | ATCCTGCCCTTCAGCCCATGCTCCTCCTGT |
| 46 | 47590 | CCTAGCTGGGAGCGCGCGGTGTGCGTTGCC |
| 47 | 46727 | AGTGAAATGCTAGCTACGCCGTTCTCCTTC |
| 48 | 45352 | TCCGATCCTTTGGACTCCGCGGCGGCTCGT |
| 49 | 44284 | ATTCTCTCACCTCGCACCCAGGCGGGCGCC |

(continued)

| SEQ ID NO. | Expression level (mRFP) | 3' UTR sequence |
|---|---|---|
| 50 | 42824 | TAGTACAAATTTGCTAACATAAATACAATC |
| 51 | 41525 | ACGAACCTTTCCACTCCCCAATTCTTAAGT |
| 52 | 41331 | TCTTCCTACTCTCTCCTACTGTCTCCTTTC |
| 53 | 39852 | TCGGAAACTACGTCTTCGTCATAAACCCTC |
| 54 | 36239 | TAACTAATACATACCACTGAGATCTCCTCC |
| 55 | 35361 | TAGCACACAAAACGAAATAAATCAACCAAG |
| 56 | 30280 | AATATACCAAGCAAATACAGGTGACGCAAT |
| 57 | 18374 | TAATACCAAACTAAGCTTAAAGAAAGCAAC |

Table 2. Raw expression levels and sequence results for GFPuv.

| SEQ ID NO. | Expression level (GFPuv) | 3' UTR sequence |
|---|---|---|
| 58 | 38257 | CTTTAGTCTCGGTATACTCTTCTGTTTTCG |
| 59 | 34595 | AGTTGTCCGTGCGTCTCTTAAACTGGTAAA |
| 60 | 34401 | CAAGTAAGCTTGAGGCCTAACTACAACAAA |
| 61 | 33026 | ACCTTACTTCGCTTAAAACTCTGTATCTAA |
| 62 | 32869 | CGAACGGGTTAGACGTATATAAACTGAAAA |
| 63 | 32795 | GACCTCGCCCACCCAAGTTGCACCTATGTA |
| 64 | 32143 | CAATTGAGCACACCGGACCCAACCACTACA |
| 65 | 31964 | TCCATGTCCCGCACCTTTCCCTATTCTACT |
| 66 | 31957 | CCTGATCAAATTATGAAATAAACCTCTGAA |
| 67 | 31923 | CCTGTAAAGCGAACGAGCAAAACTCATACA |
| 68 | 31792 | CGCCACCAGACATGCCCGTTCTTACTAACC |
| 69 | 31775 | CCCTCCACTTAATGGCAAGCAGTCCTTCCA |
| 70 | 31770 | ACCGGGCCCCACCCCGTTTGCAATACCTCA |
| 71 | 31694 | CTCATTTGCTACTCACTTTATAGCACTGTA |
| 72 | 31690 | TCTTGAATGTTAAGCATGCAGTTAATACAG |
| 73 | 31668 | AGCCTGACCTTGATTATGAGAGTGAACAAA |
| 74 | 31649 | CTTCACGTTCACGCCTTTACAATTGATTTA |
| 75 | 31542 | CCCCTATCATCGCTTCTGGTACATCACCTA |
| 76 | 31467 | AGGGCCTTCCCTTCCCTACCTCTGTCCAAC |
| 77 | 31345 | ATCTCATTCGTGATTGTATATGATTGAGTC |
| 78 | 31229 | AGGTTCGTTGGTGTTTATAACACTTTGTTT |
| 79 | 31106 | CTCAGAGCCCCTGTAGTCAGCACTTTGCCC |
| 80 | 31043 | ACCACACGATTAAATCCCAGAAACATCATA |
| 81 | 30955 | ACTAAGCTTTACATAAGGCTGATTGTGCAC |
| 82 | 30935 | ACACGTTTCCGGTGTTGGCCCATCACGATA |
| 83 | 30902 | ACATGAGCGGAATCGCTAACTAAGTTAAAC |

(continued)

| SEQ ID NO. | Expression level (GFPuv) | 3' UTR sequence |
|---|---|---|
| 84 | 30835 | AATTGTCGATGTATGCTAAAACTTCAAATT |
| 85 | 30833 | CTCAATCCATAGACCAATCCAACCAATTCT |
| 86 | 30812 | ATCGATATTCCGCTAGATATATGTTCATTT |
| 87 | 30601 | CTGGGCGTCCAACTAAGGCCCCACGGACCT |
| 88 | 30495 | ATCGACACCCTACCGGACAACTTTGTCTGC |
| 89 | 30487 | TCCTTTCTGCAGTAAGAAGTAAACGAGAAT |
| 90 | 30410 | TCCATGTCCCGCACCTTTCCCTATTCTACT |
| 91 | 30372 | CTCAGAGCCCCTGTAGTCAGCACTTTGCCC |
| 92 | 30331 | TAGCTAGGTTGCTTGACAATCTGTCCCTTC |
| 93 | 30163 | AATTTTAGGCTATTACGAAGACTTGTTATT |
| 94 | 29730 | CTGAAATCACTAATGTTTCGGTAAAACGCT |
| 95 | 29702 | TCCTGCCGCGCGCACGGTTGGGCAACGGCA |
| 96 | 29652 | TCCAGGCAAAGGCACCCTTCGAAACGCACT |
| 97 | 29584 | ACTAGTTCTGGTATCATTAGGTCTAGTTGC |
| 98 | 29553 | AATCTTCCTGCACTCACTAGCCGTCTTATT |
| 99 | 29152 | CTCACAGACCTTCCTCACCCATCTGACTCC |
| 100 | 29076 | CTGAAATCACTAATGTTTCGGTAAAACGCT |
| 101 | 28840 | TCGCCTTCAACAGGGCCTATCCAGTACCCC |
| 102 | 28709 | ATCAGACACCTTATGACTACCAGTAAAGTC |
| 103 | 28626 | CTTGTTGAGTGGTGCCTCGGGGAGCGAGGG |
| 104 | 28293 | TGCCTGCGCGGCGCCCCGGGCGACAGGCCC |
| 105 | 28148 | CCACGGTGAAGCTAATCACCTCCCGGTGCC |
| 106 | 27866 | CCAAAGCCATGGCTGTCTGCAACCAAAGAC |
| 107 | 27341 | CCGCTCTTCAGAGCGCCTAATCTCCGAGCC |
| 108 | 27157 | ATCAATGGTTAGCGTAATCGACACTATACC |
| 109 | 26959 | TTCTTTGCGTTCTAACATGTTGCGTATACT |
| 110 | 26574 | CATCACAGACCCAAGAGCCGCAAAATCGTC |
| 111 | 26496 | TCATACCTAGCTCCTAGTACATTCCCCGCG |
| 112 | 26413 | TTAGCAAACTTAGCCGATTAATAGAACGAT |
| 113 | 22798 | ACCCGAGAACCTCCCCGCCCGCTCCCCACT |
| 114 | 22104 | TCGAGGAGGGTGTGGCGAAATCTCAGTGCT |
| 115 | 22018 | CGATTAAACGCCATAGCAGCGTGGGGCGGC |
| 116 | 21922 | CGGGACCCACAGGGCCCAGTACCCTGTGGG |
| 117 | 21553 | CGGGCGTAAGGGCGCGTGCGGCGGTGTGTG |
| 118 | 21261 | ACAACAGAGTCCGACCGAGAGGGGCCGAGT |
| 119 | 21059 | CCAGTTGTGCGTCACCTTGTCATTGTTTGT |
| 120 | 20677 | ACTACAGCTATGCTCCGAATCTACAGGAAA |
| 121 | 20622 | TCTTTCGGCTCGTGGCGCGCGCTCCCCGCG |

(continued)

| SEQ ID NO. | Expression level (GFPuv) | 3' UTR sequence |
|---|---|---|
| 122 | 19503 | TGGTTTCTACCGAGCGGCCGGCTCCCTCGC |
| 123 | 18815 | TCGTGCTCGGCATATGCGGGCGGGGCAATA |
| 124 | 17980 | GCGTGTTCCTATTTCCATTCATGTAGGTAT |
| 125 | 16654 | CCGGCGGGTGTGCGCGCGTGTTCCGCCGCT |
| 126 | 16098 | TCGCAGAGCTGTATTAAGCCCATTGCAATC |
| 127 | 15087 | CACCATCCACACGTCGAAGCATATGTTAAT |
| 128 | 13991 | CGCGCGTGCCAGTGTGGGTGGGCGGGCGGC |
| 129 | 13722 | CTCCCCACATTAGACCTTAGCGGGAACGTC |

Example 2: exchange of 3' UTRs

Methods

A selection of 3' UTR sequences, 5 from the mRFP library (SEQ ID NOs 2, 9, 36, 53, 57) and 5 from the GFPuv library (SEQ ID NOs 58, 64, 112, 122, 129) were obtained from Integrated DNA Technologies (Coralville, Iowa) as phosphorylated oligonucleotides with their associated complementary part to allow annealing of the oligonucleotides into double stranded DNA with 3 base pair overhangs on both sides complementary to the 3 base pair overhang of the plasmid, which was digested with SapI (NEB, R0569) according to standard protocol (NEB). The plasmids contained either GFPuv (SEQ ID NO. 136), mRFP (SEQ ID NO. 137) or LacZ (SEQ ID NO. 138) as a reporter protein to allow quantification of the influence of the 3' UTR (Fig. 5). The double stranded DNA was ligated into the digested vector to create the testing plasmids using standard procedures and the resulting plasmids electroporated into prokaryotic cells ((ThermoFisher Scientific). To ensure an equal growth start, bacteria harbouring different constructs were grown in a pre-culture of 200 $\mu$L M9TG medium (1x M9 salts (Sigma; Merck KGaA, Darmstadt, Germany), 10 g/l tryptone (Oxoid Ltd, Thermo Fisher Microbiology Basingstoke, UK), 5 g/l glycerol (Acros Organics. Fisher Scientific, New Jersey, USA), supplemented with 50 mg/L kanamycin in a 96 wells 2 mL Masterblock (Greiner). The Masterblock was covered with a gas-permeable membrane and incubated overnight at 37°C. The pre-cultures were diluted 10000x in fresh 200 $\mu$L M9TG and grown in the same way as the pre-cultures. The cultures (and control (blank) medium) were then cooled down to room temperature and diluted 5x in 1x phosphate buffered saline (PBS) pH 7.4. Finally, 100 $\mu$L of the dilution was measured with a BioTek Synergy MX microplate (Biotek, USA) reader at excitation 395/20 nm, emission 508/20 nm, gain 75 (GFPuv), and excitation 584/9 nm, emission 607/9 nm, gain 100 (mRFP). Fluorescence was calculated as raw fluorescence per OD600 for 100 $\mu$L 5x dilution using a plate reader. Data were corrected OD. Auto-fluorescence, estimated by introduction of a frameshift mutation in a GFP-expressing construct, was subtracted and samples were normalised by dividing by one of the wild-type (WT) samples. To measure LacZ activity, cultures were diluted 5x in permeabilisation solution (Zhang and Bremer, 1995. J Biol Chem 270: 11181-11189) and 20 $\mu$L of culture was mixed with 80 $\mu$L of permeabilisation solution (100 mM $Na_2HPO_4$, 20 mM KCl, 2 mM $MgSO_4$, 0.8 g/L cetyl trimethylammonium bromide (CTAB), 0.4 g/L sodium deoxycholate and 5.4 mL/L $\beta$-mercaptoethanol) and incubated at 30°C for 30 min. 600 $\mu$L of pre-warmed substrate solution (60 mM $Na_2HPO_4$, 40 mM $NaH_2PO_4$, 1 g/L o-nitrophenyl-$\beta$-D-galactopyranoside (ONPG) and 2.7 mL/L $\beta$-mercaptoethanol) was added and incubated at 30°C until colour had developed. 700 $\mu$L of stop solution (1 M $Na_2CO_3$) was added to quench the reaction. The reaction was filtered through a 0.2 $\mu$M filter and measured in a spectrophotometer at 420 nm in a 1 cm cuvette. Activity was calculated as:

$$LacZ = \frac{A_{420}}{t} * \frac{Vtotal}{Vculture * OD_{600}}$$

Results

5 representative sequences (Fig. 3A; SEQ ID NOs 5, 9, 36, 53, 57) of the mRFP library and 5 representative sequences (Fig 3A; SEQ ID NOs 58, 64, 112, 122, 129) of the GFPuv library were cloned in a pUC-based plasmid with each of GFPuv, mRFP or LacZ as a reporter protein (protein of interest). The associated expression value is given as the measured signal over the mean of the signal (Fig 3A). Truncations were made of a sequence (SEQ ID NO. 64) in an attempt to discover an essential part of the sequence that contributes to the positive effect on expression but no specific region was discovered (Table 3: SEQ ID NO.138-143). The expression correlations between the different reporters show correlations indicating that the effect the 3' UTR has on translation is mostly ORF independent (Figure 3B-D).

Table 3: truncations of SEQ ID NO. 64

| SEQ ID NO. | 3' UTR sequence |
|---|---|
| 138 | CAATTGAGCACACCGGACCCAACCA |
| 139 | CAATTGAGCACACCGGACCC |
| 140 | CAATTGAGCACACCG |
| 141 | GAGCACACCGGACCCAACCACTACA |
| 142 | CACCGGACCCAACCACTACA |
| 143 | GACCCAACCACT ACA |

Example 3: exchange of the intergenic regions

Methods

A selection of 3' UTR sequences, 2 from the mRFP library (SEQ ID NOs 57 and 58) were ordered (IDT) as phosphorylated oligonucleotides with their associated complementary strand to allow annealing of the oligonucleotides into double stranded DNA with 3 base pair overhangs on both sides complementary to the 3 base pair overhang of the plasmid, which was digested with SapI (NEB, R0569) according to the manufacturer's instructions. The plasmids contained both GFPuv (SEQ ID NO. 136) and mRFP (SEQ ID NO. 137) as reporter proteins to allow quantification of the influence of the intergenic region (IGR) between mRFP and GFPuv. Two plasmids were used, comprising the order GFPuv-intergenic region-mRFP and comprising the reversed order mRFP-intergenic region- GFPuv. The intergenic region comprised the PSAT sequences and, where indicated, the terminator guanidine-cytidine (GC)-rich hairpin (stem) sequences, but not the terminator stretch of uridine residues. The double stranded DNA was ligated into the digested vector to create the testing plasmids using standard procedures. To ensure an equal growth start, bacteria harbouring different constructs were grown in a pre-culture of 200 µL M9TG medium (1x M9 salts (Sigma), 10 g/l tryptone (Oxoid), 5 g/l glycerol (Acros), supplemented with 50 mg/L kanamycin in a 96 wells 2 mL Masterblock (Greiner). The Masterblock was covered with a gas-permeable membrane and incubated overnight at 37°C. The pre-cultures were diluted 10.000x in fresh 200 µL M9TG and grown overnight in the same way as the pre-cultures. The cultures and control (blank) medium were then cooled down to room temperature and diluted 5x in 1x phosphate buffered saline (PBS) pH 7.4. Finally, 100 µL of the dilution was measured with a BioTek Synergy MX microplate (Biotek, USA) reader at excitation 395/20 nm, emission 508/20 nm, gain 75 (GFPuv), and excitation 584/9 nm, emission 607/9 nm, gain 100 (mRFP). Fluorescence was calculated as raw fluorescence per OD600 for 100 µL 5x dilution. Auto-fluorescence, estimated by introduction of a frameshift in a GFP was subtracted and samples were normalised by dividing by one of the wild-type (WT) samples.

Results

2 sequences on either end of the translation efficiency spectrum (see Fig. 3A; SEQ ID NOs 57 and 58) were cloned in a vector between GFPuv and mRFP, which both served as a reporter protein (protein of interest). The associated expression value is provided as the measured signal over the mean of the signal (Fig 6). It can be concluded that exchanging the intergenic region within an operon can modulate both the total translation of the operon, as well as individual ORFs.

**Claims**

1.  A method of modulating gene expression in prokaryotes, comprising

  (i) selecting a prokaryotic expression cassette expressing a protein of interest, said expression cassette comprising a promoter, a 5' UTR containing a ribosome binding site, a coding sequence for a protein of interest including a stop codon, a 3' untranslated region (UTR) and a terminator;
  (ii) replacing the nucleotide sequence of the 3' UTR between the stop codon of the protein of interest and the terminator with a randomized nucleotide sequence to generate a library of expression cassettes expressing the protein of interest;

(iii) expressing the library of expression cassettes expressing the protein of interest in a prokaryote; and

(iv) selecting an expression cassette with modulated expression of the protein of interest, preferably with increased expression of the protein of interest, when compared to the selected expression cassette under (i).

2. The method according to claim 1, wherein the randomized nucleotide sequence is 20-100 nucleotides, preferably about 25-50 nucleotides such as about 30 nucleotides.

3. A method of modulating gene expression in prokaryotes, comprising

(i) selecting an expression cassette expressing a protein of interest;

(ii) replacing the 3' untranslated region (UTR) between stop codon and terminator for a nucleotide sequence depicted in Table 1;

(iii) expressing the resulting expression cassettes expressing the protein of interest in a prokaryote; and

(iv) selecting an expression cassette with modulated expression of the protein of interest, preferably with increased expression of the protein of interest, when compared to the selected expression cassette under (i).

4. A prokaryotic expression cassette, comprising an expression cassette for expression of a protein of interest, said expression cassette comprising a promoter, a poly-linker, a 3' untranslated region (UTR) comprising a nucleotide sequence as depicted in Table 1, and a terminator.

5. The prokaryotic expression cassette according to claim 4, wherein the terminator is a Rho-independent terminator.

6. The prokaryotic expression cassette according to claim 4 or 5, wherein the terminator comprises the sequence 5'-CCCCGCTTCGGCGGGGTTTTTTT.

7. A method of expressing a protein of interest in a prokaryotic cell, comprising
providing a prokaryotic expression cassette according to any one of claims 4-6;
inserting a nucleotide sequence encoding a protein of interest in between the promoter and the 3' untranslated region (UTR) such that the promoter directs expression of the protein of interest;
transforming a prokaryotic cell with the prokaryotic expression cassette comprising the nucleotide sequence encoding a protein of interest; and
expressing the protein of interest.

8. The method according to claim 7, wherein the nucleotide sequence encoding a protein of interest is codon optimized for expression in said prokaryotic cell.

9. A prokaryotic cell, comprising the prokaryotic expression cassette according to any one of claims 4-6 in which a nucleotide sequence encoding a protein of interest is inserted in between the promoter and the 3' untranslated region (UTR) such that the promoter directs expression of the protein of interest.

10. The prokaryotic cell according to claim 9, which is *Escherichia coli.*

11. The prokaryotic cell according to claim 9 or 10, wherein the nucleotide sequence encoding a protein of interest is codon optimized for expression in said prokaryotic cell.

12. A method of expressing a protein of interest in a prokaryotic cell, comprising providing the prokaryotic cell according to any one of claims 9-11; and expressing the protein of interest.

13. A method of producing a protein of interest in a prokaryotic cell, comprising providing the prokaryotic cell according to any one of claims 9-11; expressing the protein of interest thus producing the protein of interest.

14. The method according to claim 13, further comprising at least partly purifying the protein of interest.

15. The method according to any one of claims 12-14, wherein the protein of interest is tagged.

Figure 1

1. Starting material

*Primer*

5' AGCGTAGCTGAATT GGTCTC 3'

*ssDNA*

3' TCGCATCGACTTAA CCAGAGT TATT NNNNNNNNNNNNNNNNNNNNNNNNNNNNNN GGGG ACTCTGG 5'
                        BsaI              30 degenerate nucleotides              BsaI

2. Generated dsDNA via PCR with ssDNA as template and the Primer.
Digested the dsDNA with BsaI. Digested the plasmid DNA with BsaI.

*Digested dsDNA*

ATAANNNNNNNNNNNNNNNNNNNNNNNNNNNNNNNNNN
      NNNNNNNNNNNNNNNNNNNNNNNNNNNNNNNNNNGGGG

*Digested plasmid DNA*

The stop codon
of the GOI

The beginning of
the terminator

3. Inserted the digested dsDNA into the digested plasmid DNA via a ligation reaction

*Plasmid DNA with degenerate 30 nucleotide region*

----ATAANNNNNNNNNNNNNNNNNNNNNNNNNNNNNNNNNNCCCC----
----TATTNNNNNNNNNNNNNNNNNNNNNNNNNNNNNNNNNNGGGG----

Figure 2

Figure 3

Figure 3 (continued)

Figure 4

| SEQ ID NO. | Description | Sequence |
|---|---|---|
| 130 | Single stranded DNA block | GGTCTCAGGGGNNNNNNNNNNNNNNNNNNNNNNNNNNNNNNNNNNTTAATGAGACCAATTCAGCTACGCT |
| 131 | Primer for DNA block | AGCGTAGCTGAATTGGTCTC |
| 132 | Backbone. Containing a P15A origin, Kanamycin resistance gene, and mRFP driven by a bla promoter. Additional nonsense sequence between stop codon and terminator. | GTGCTTTCTATGAGTCGTTGCTGCATAACTTCAAATATGTATCCGCTCATGAGACAATGGGCCCAAGTTCACTTAAAAAGGAGATCAACAATGAAAGCAATTTTCGTACTGAAACATCTTAATCATGCAGGGGAGGGTTTCTAATGGCGAGTAGCGAAGACGTTATCAAAGAGTTCATGCGTTTCAAAGTTCGTATGGAAGGTTCCGTTAACGGTCACGAGTTCGAAATCGAAGGTGAAGGTGAAGGTCGTCCGTACGAAGGTACCCAGACCGCTAAACTGAAAGTTACCAAAGGTGGTCCGCTGCCGTTCGCTTGGGACATCCTGTCCCCGCAGTTCCAGTACGGTTCCAAAGCTTACGTTAAACACCCGGCTGACATCCCGGACTACCTGAAACTGTCCTTCCCGGAAGGTTTCAAATGGGAACGTGTTATGAACTTCGAAGACGGTGGTGTTGTTACCGTTACCCAGGACTCCTCCCTGCAAGACGGTGAGTTCATCTACAAAGTTAAACTGCGTGGTACCAACTTCCCGTCCGACGGTCCGGTTATGCAGAAAAAAACCATGGGTTGGGAAGCTTCCACCGAACGTATGTACCCGGAAGACGGTGCTCTGAAAGGTGAAATCAAAATGCGTCTGAAACTGAAAGACGGTGGTCACTACGACGCTGAAGTTAAAACCACCTACATGGCTAAAAAACCGGTTCAGCTGCCGGGTGCTTACAAAAACCGACATCAAACTGGACATCACCTCCCACAACGAAGACTACACCATCGTTGAACAGTACGAACGTGCTGAAGGTCGTCACTCCACCGGTGCTTAATGAGACCTCCCAATTCTTGTTGAATTAGATGGTGATGTTAATGGGCACAAATTTTCTGTCAGTGGAGAGGGTGAAGGTGATGCAACATACGGAAAACTTACCCTTAAATTTATTTGCACTACTGGAAAACTACCTGTTCCATGGCCAACACTTGTCACTACTTTCTCTTATGGTGTTCAATGCTTTTCCCGTTATCCGGATCACATGAAACGGCATGACTTTTTCAAGAGTGCCATGCCCGAAGGTTATGTACAGGAACGCACTATATCTTTCAAAGATGACGGGAACTACAAGACGCGTGCTGAAGTCAAGTTTGAAGGTGATACCCTTGTTAATCGTATCGAGTTAAAAGGTATTGATTTTAAAGAAGATGGAAACATTCTCGGACACAAACTGGAGTACAACTATAACTCACACAATGTATACATCACGGCAGACAAACAAAAGAATGGAATCAGGTCTCTCCCCGCTTCGGCGGGGTTTTTTTGCAAGGGATCGGTTGTCGAGTAAGGATCTCCAGGCATCCTGTTGTTTGTCGGTGAACGCTCTCTACTAGAGTCACACTGGCTCACCTTCGGGTGGGCCTTTCTGCGTTTATAGGATCCTAACTCGAGCCTAGGGATATATTCCGCTTCCTCGCTCACTGACTCGCTACGCTCGGTCGTTCGACTGCGGCGAGCGGAAATGGCTTACGAACGGGGCGGAGATTTCCTGGAAGATGCCAGGAAGATACTTAACAGGGAACTGAGAGGGCCGCGGCAAAGCCGTTTTTCCATAGGCTCCGCCCCCTGACAAGCATCACGAAATCTGACGCTCAAATCAGTGGTGGCGAAACCCGACAGGACTATAAAGATACCAGGCGTTTCCCCCT |

| | | |
|---|---|---|
| | | GGCGGCTCCCTCGTGCGCTCTCCTGTTCCTGCCTTTCGGTT<br>TACCGGTGTCATTCCGCTGTTATGGCCGCGTTTGTCTCATT<br>CCACGCCTGACACTCAGTTCCGGGTAGGCAGTTCGCTCCA<br>AGCTGGACTGTATGCACGAACCCCCCGTTCAGTCCGACCG<br>CTGCGCCTTATCCGGTAACTATCGTCTTGAGTCCAACCCG<br>GAAAGACATGCAAAGCACCACTGGCAGCAGCCACTGGTA<br>ATTGATTTAGAGGAGTTAGTCTTGAAGTCATGCGCCGGTT<br>AAGGCTAAACTGAAAGGACAAGTTTTGGTGACTGCGCTCC<br>TCCAAGCCAGTTACCTCGGTTCAAAGAGTTGGTAGCTCAG<br>AGAACCTTCGAAAAACCGCCCTGCAAGGCGGTTTTTTCGT<br>TTTCAGAGCAAGAGATTACGCGCAGACCAAAACGATCTCA<br>AGAAGATCATCTTATTAATCAGATAAAATATTTCTAGATTT<br>CAGTGCAATTTATCTCTTCAAATGTAGCACCTGAAGTCAGC<br>CCCATACGATATAAGTTGTTACTAGTGCTTGGATTCTCACC<br>AATAAAAAACGCCCGGCGGCAACCGAGCGTTCTGAACAAA<br>TCCAGATGGAGTTCTGAGGTCATTACTGGATCTATCAACA<br>GGAGTCCAAGCGAGCTCTCGAACCCCAGAGTCCCGCTCAG<br>AACAACTCGTCAAGAAGGCGATAGAAGGCGATGCGCTGCG<br>AATCGGGAGCGGCGATACCGTAAAGCACGAGGAAGCGGT<br>CAGCCCATTCGCCGCCAAGCTCTTCAGCAATATCACGGGT<br>AGCCAACGCTATGTCCTGATAGCGGTCCGCCACACCCAGC<br>CGGCCACAGTCGATGAATCCAGAAAAGCGGCCATTTTCCA<br>CCATGATATTCGGCAAGCAGGCATCGCCATGGGTCACGAC<br>GAGATCCTCGCCGTCGGGCATGCGCGCCTTGAGCCTGGCG<br>AACAGTTCGGCTGGCGCGAGCCCCTGATGCTCTTCGTCCA<br>GATCATCCTGATCGACAAGACCGGCTTCCATCCGAGTACG<br>TGCTCGCTCGATGCGATGTTTCGCTTGGTGGTCGAATGGG<br>CAGGTAGCCGGATCAAGCGTATGCAGCCGCCGCATTGCAT<br>CAGCCATGATGGATACTTTCTCGGCAGGAGCAAGGTGAGA<br>TGACAGGAGATCCTGCCCCGGCACTTCGCCCAATAGCAGC<br>CAGTCCCTTCCCGCTTCAGTGACAACGTCGAGCACAGCTG<br>CGCAAGGAACGCCCGTCGTGGCCAGCCACGATAGCCGCGC<br>TGCCTCGTCCTGCAGTTCATTCAGGGCACCGGACAGGTCG<br>GTCTTGACAAAAAGAACCGGGCGCCCCTGCGCTGACAGCC<br>GGAACACGGCGGCATCAGAGCAGCCGATTGTCTGTTGTGC<br>CCAGTCATAGCCGAATAGCCTCTCCACCCAAGCGGCCGGA<br>GAACCTGCGTGCAATCCATCTTGTTCAATCATGCGAAACG<br>ATCCTCATCCTGTCTCTTGATCAGATCATGATCCCCTGCGC<br>CATCAGATCCTTGGCGGCAAGAAAGCCATCCAGTTTACTTT<br>GCAGGGCTTCCCAACCTTACCAGAGGGCGCCCCAGCTGGC<br>AATTCCGACGTCGAATTCAAAAGATCTTAAGTAAGTAAGA<br>GTATACGTATATCGGCTAATAACGTATTAAGGCGCTTCGG<br>CGCCTTTTTTTATGGGGGTATTTTCATCCCAATCCACACGT<br>CCAACCCACAGCAAACACCACGTCGACCCTATCAGCTGC |
| 133 | Backbone.<br>Containing a<br>P15A origin,<br>Kanamycin<br>resistance<br>gene, and<br>GFPuv driven<br>by a bla | GTGCTTTCTATGAGTCGTTGCTGCATAACTTCAAATATGTA<br>TCCGCTCATGAGACAATGGGCCCAAGTTCACTTAAAAAGG<br>AGATCAACAATGAAAGCAATTTTCGTACTGAAACATCTTAA<br>TCATGCAGGGGAGGGTTTCTAATGAGTAAAGGAGAAGAAC<br>TTTTCACTGGAGTTGTCCCAATTCTTGTTGAATTAGATGGT<br>GATGTTAATGGGCACAAATTTTCTGTCAGTGGAGAGGGTG<br>AAGGTGATGCAACATACGGAAAACTTACCCTTAAATTTATT<br>TGCACTACTGGAAAACTACCTGTTCCATGGCCAACACTTGT |

| | promoter. Additional nonsense sequence between stop codon and terminator. | CACTACTTTCTCTTATGGTGTTCAATGCTTTTCCCGTTATC CGGATCACATGAAACGGCATGACTTTTTCAAGAGTGCCAT GCCCGAAGGTTATGTACAGGAACGCACTATATCTTTCAAA GATGACGGGAACTACAAGACGCGTGCTGAAGTCAAGTTTG AAGGTGATACCCTTGTTAATCGTATCGAGTTAAAAGGTATT GATTTTAAAGAAGATGGAAACATTCTCGGACACAAACTGG AGTACAACTATAACTCACACAATGTATACATCACGGCAGAC AAACAAAGAATGGAATCAAAGCTAACTTCAAAATTCGCC ACAACATTGAAGATGGATCCGTTCAACTAGCAGACCATTAT CAACAAATACTCCAATTGGCGATGGCCCTGTCCTTTTACC AGACAACCATTACCTGTCGACACAATCTGCCCTTTCGAAAG ATCCCAACGAAAAGCGTGACCACATGGTCCTTCTTGAGTTT GTAACTGCTGCTGGGATTACACATGGCATGGATGAGCTCT ACAAATAATGAGACCTCCCAATTCTTGTTGAATTAGATGGT GATGTTAATGGGCACAAATTTTCTGTCAGTGGAGAGGGTG AAGGTGATGCAACATACGGAAAACTTACCCTTAAATTTATT TGCACTACTGGAAAACTACCTGTTCCATGGCCAACACTTGT CACTACTTTCTCTTATGGTGTTCAATGCTTTTCCCGTTATC CGGATCACATGAAACGGCATGACTTTTTCAAGAGTGCCAT GCCCGAAGGTTATGTACAGGAACGCACTATATCTTTCAAA GATGACGGGAACTACAAGACGCGTGCTGAAGTCAAGTTTG AAGGTGATACCCTTGTTAATCGTATCGAGTTAAAAGGTATT GATTTTAAAGAAGATGGAAACATTCTCGGACACAAACTGG AGTACAACTATAACTCACACAATGTATACATCACGGCAGAC AAACAAAGAATGGAATCAGGTCTCTCCCCGCTTCGGCGG GGTTTTTTTGCAAGGGATCGGTTGTCGAGTAAGGATCTCC AGGCATCCTGTTGTTTGTCGGTGAACGCTCTCTACTAGAGT CACACTGGCTCACCTTCGGGTGGGCCTTTCTGCGTTTATA GGATCCTAACTCGAGCCTAGGGATATATTCCGCTTCCTCG CTCACTGACTCGCTACGCTCGGTCGTTCGACTGCGGCGAG CGGAAATGGCTTACGAACGGGGCGGAGATTTCCTGGAAGA TGCCAGGAAGATACTTAACAGGGAAGTGAGAGGGCCGCG GCAAAGCCGTTTTTCCATAGGCTCCGCCCCCTGACAAGC ATCACGAAATCTGACGCTCAAATCAGTGGTGGCGAAACCC GACAGGACTATAAAGATACCAGGCGTTTCCCCCTGGCGGC TCCCTCGTGCGCTCTCCTGTTCCTGCCTTTCGGTTTACCGG TGTCATTCCGCTGTTATGGCCGCGTTTGTCTCATTCCACGC CTGACACTCAGTTCCGGGTAGGCAGTTCGCTCCAAGCTGG ACTGTATGCACGAACCCCCCGTTCAGTCCGACCGCTGCGC CTTATCCGGTAACTATCGTCTTGAGTCCAACCCGGAAAGA CATGCAAAGCACCACTGGCAGCAGCCACTGGTAATTGAT TTAGAGGAGTTAGTCTTGAAGTCATGCGCCGGTTAAGGCT AAACTGAAAGGACAAGTTTTGGTGACTGCGCTCCTCCAAG CCAGTTACCTCGGTTCAAAGAGTTGGTAGCTCAGAGAACC TTCGAAAAACCGCCCTGCAAGGCGGTTTTTTCGTTTTCAGA GCAAGAGATTACGCGCAGACCAAAACGATCTCAAGAAGAT CATCTTATTAATCAGATAAAATATTTCTAGATTTCAGTGCA ATTTATCTCTTCAAATGTAGCACCTGAAGTCAGCCCCATAC GATATAAGTTGTTACTAGTGCTTGGATTCTCACCAATAAAA AACGCCCGGCGGCAACCGAGCGTTCTGAACAAATCCAGAT GGAGTTCTGAGGTCATTACTGGATCTATCAACAGGAGTCC AAGCGAGCTCTCGAACCCCAGAGTCCCGCTCAGAAGAACT |

| | | |
|---|---|---|
| | | CGTCAAGAAGGCGATAGAAGGCGATGCGCTGCGAATCGG GAGCGGCGATACCGTAAAGCACGAGGAAGCGGTCAGCCC ATTCGCCGCCAAGCTCTTCAGCAATATCACGGGTAGCCAA CGCTATGTCCTGATAGCGGTCCGCCACACCCAGCCGGCCA CAGTCGATGAATCCAGAAAAGCGGCCATTTTCCACCATGA TATTCGGCAAGCAGGCATCGCCATGGGTCACGACGAGATC CTCGCCGTCGGGCATGCGCGCCTTGAGCCTGGCGAACAGT TCGGCTGGCGCGAGCCCCTGATGCTCTTCGTCCAGATCAT CCTGATCGACAAGACCGGCTTCCATCCGAGTACGTGCTCG CTCGATGCGATGTTTCGCTTGGTGGTCGAATGGGCAGGTA GCCGGATCAAGCGTATGCAGCCGCCGCATTGCATCAGCCA TGATGGATACTTTCTCGGCAGGAGCAAGGTGAGATGACAG GAGATCCTGCCCCGGCACTTCGCCCAATAGCAGCCAGTCC CTTCCCGCTTCAGTGACAACGTCGAGCACAGCTGCGCAAG GAACGCCCGTCGTGGCCAGCCACGATAGCCGCGCTGCCTC GTCCTGCAGTTCATTCAGGGCACCGGACAGGTCGGTCTTG ACAAAAAGAACCGGGCGCCCCTGCGCTGACAGCCGGAACA CGGCGGCATCAGAGCAGCCGATTGTCTGTTGTGCCCAGTC ATAGCCGAATAGCCTCTCCACCCAAGCGGCCGGAGAACCT GCGTGCAATCCATCTTGTTCAATCATGCGAAACGATCCTCA TCCTGTCTCTTGATCAGATCATGATCCCCTGCGCCATCAGA TCCTTGGCGGCAAGAAAGCCATCCAGTTTACTTTGCAGGG CTTCCCAACCTTACCAGAGGGCGCCCCAGCTGGCAATTCC GACGTCGAATTCAAAAGATCTTAAGTAAGTAAGAGTATAC GTATATCGGCTAATAACGTATTAAGGCGCTTCGGCGCCTTT TTTTATGGGGGTATTTTCATCCCAATCCACACGTCCAACGC ACAGCAAACACCACGTCGACCCTATCAGCTGC |
| 134 | Amplification primer 1 | AATTCCGACGTCGAATTC |
| 135 | Amplification primer 2 | GCGTCAGATTTCGTGATG |

Figure 5

| SEQ ID NO. | Description | Sequence |
|---|---|---|
| 136 | Backbone. Containing a P15A origin, Kanamycin resistance gene, chloramphenicol resistance gene and GFPuv driven by a tac promoter. The backbone sequence contains 2 SapI sites that excise the chloramphenicol resistance gene and leave the overhangs for insertion of the 3' UTR. | TCTAGATTTCAGTGCAATTTATCTCTTCAAATGTAGCACC TGAAGTCAGCCCCATACGATATAAGTTGTAATTCGGTAC CCCGCTTCGGCGGGGTTTTTTCAAGTTCAAATATGTATCC GCTCATGAGACAATGTGTGGGGAGACCACAACGGTTTCC CTCTAGAAATAATTTTGTTTAACTATAAGAAGGAGATATA CATATGAGTAAAGGAGAAGAACTTTTCACTGGAGTTGTC CCAATTCTTGTTGAATTAGATGGTGATGTTAATGGGCAC AAATTTTCTGTCAGTGGAGAGGGTGAAGGTGATGCAACA TACGGAAAACTTACCCTTAAATTTATTTGCACTACTGGAA AACTACCTGTTCCATGGCCAACACTTGTCACTACTTTCTC TTATGGTGTTCAATGCTTTTCCCGTTATCCGGATCACATG AAACGGCATGACTTTTTCAAGAGTGCCATGCCCGAAGGT TATGTACAGGAACGCACTATATCTTTCAAAGATGACGGG AACTACAAGACGCGTGCTGAAGTCAAGTTTGAAGGTGAT ACCCTTGTTAATCGTATCGAGTTAAAAGGTATTGATTTTA AAGAAGATGGAAACATTCTCGGACACAAACTGGAGTACA ACTATAACTCACACAATGTATACATCACGGCAGACAAAC AAAAGAATGGAATCAAAGCTAACTTCAAAATTCGCCACA ACATTGAAGATGGATCCGTTCAACTAGCAGACCATTATC AACAAAATACTCCAATTGGCGATGGCCCTGTCCTTTTACC AGACAACCATTACCTGTCGACACAATCTGCCCTTTCGAA AGATCCCAACGAAAAGCGTGACCACATGGTCCTTCTTGA GTTTGTAACTGCTGCTGGGATTACACATGGCATGGATGA GCTCTACAAATAATGAAGAGCGATCGGCACGTAAGAGGT TCCAACTTTCACCATAATGAAATAAGATCACTACCGGGC GTATTTTTTGAGTTATCGAGATTTTCAGGAGCTAAGGAA GCTAAAATGGAGAAAAAATCACTGGATATACCACCGTT GATATATCCCAATGGCATCGTAAAGAACATTTTGAGGCA TTTCAGTCAGTTGCTCAATGTACCTATAACCAGACCGTTC AGCTGGATATTACGGCCTTTTTAAAGACCGTAAAGAAAA ATAAGCACAAGTTTTATCCGGCCTTTATTCACATTCTTGC CCGCCTGATGAATGCTCATCCGGAATTCCGTATGGCAAT GAAAGACGGTGAGCTGGTGATATGGGATAGTGTTCACCC TTGTTACACCGTTTTTCCATGAGCAAACTGAAACGTTTTCA TCGCTCTGGAGTGAATACCACGACGATTTCCGGCAGTTT CTACACATATATTCGCAAGATGTGGCGTGTTACGGTGAA AACCTGGCCTATTTCCCTAAAGGGTTTATTGAGAATATGT TTTTCGTCTCAGCCAATCCCTGGGTGAGTTTCACCAGTTT TGATTTAAACGTGGCCAATATGGACAACTTCTTCGCCCC CGTTTTCACCATGGGCAAATATTATACGCAAGGCGACAA GGTGCTGATGCCGCTGGCGATTCAGGTTCATCATGCCGT CTGTGATGGCTTCCATGTCGGCAGAATGCTTAATGAATT ACAACAGTACTGCGATGAGTGGCAGGGCGGGGCGTAATT TTTTTAAGGCAGTTATTGGTGCCCTTAAACGCGCTCTTCT CCCCGCTTCGGCGGGGTTTTTTTGCAAGTGGCACTTTTC GGGGAAATGTGCGCGGAACCCCTATTTGTTTATTTTTCTA AATACATTCAAATATGTATCCGCTCATGAATTAATTCTTA |

GAAAAACTCATCGAGCATCAAATGAAACTGCAATTTATTC
ATATCAGGATTATCAATACCATATTTTTGAAAAAGCCGTT
TCTGTAATGAAGGAGAAAACTCACCGAGGCAGTTCCATA
GGATGGCAAGATCCTGGTATCGGTCTGCGATTCCGACTC
GTCCAACATCAATACAACCTATTAATTTCCCTCGTCAAA
AATAAGGTTATCAAGTGAGAAATCACCATGAGTGACGAC
TGAATCCGGTGAGAATGGCAAAAGTTTATGCATTTCTTTC
CAGACTTGTTCAACAGGCCAGCCATTACGCTCGTCATCA
AAATCACTCGCATCAACCAAACCGTTATTCATTCGTGATT
GCGCCTGAGCGAGACGAAATACGCGGTCGCTGTTAAAAG
GACAATTACAAACAGGAATCGAATGCAACCGGCGCAGGA
ACACTGCCAGCGCATCAACAATATTTTCACCTGAATCAG
GATATTCTTCTAATACCTGGAATGCTGTTTTCCCGGGGAT
CGCAGTGGTGAGTAACCATGCATCATCAGGAGTACGGAT
AAAATGCTTGATGGTCGGAAGAGGCATAAATTCCGTCAG
CCAGTTTAGTCTGACCATCTCATCTGTAACATCATTGGCA
ACGCTACCTTTGCCATGTTTCAGAAACAACTCTGGCGCA
TCGGGCTTCCCATACAATCGATAGATTGTCGCACCTGAT
TGCCCGACATTATCGCGAGCCCATTTATACCCATATAAAT
CAGCATCCATGTTGGAATTTAATCGCGGCCTAGAGCAAG
ACGTTTCCCGTTGAATATGGCTCATACTCTTCCTTTTTCA
ATATTATTGAAGCATTTATCAGGGTTATTGTCTCATGAGC
GGATACATATTTGAATGTATTTAGAAAAATAAACAAATAG
GCTGTCCCTCCTGTTCAGCTACTGACGGGGTGGTGCGTA
ACGGCAAAAGCACCGCCGGACATCAGCGCTAGCGGAGT
GTATACTGGCTTACTATGTTGGCACTGATGAGGGTGTCA
GTGAAGTGCTTCATGTGGCAGGAGAAAAAAGGCTGCACC
GGTGCGTCAGCAGAATATGTGATACAGGATATATTCCGC
TTCCTCGCTCACTGACTCGCTACGCTCGGTCGTTCGACT
GCGGCGAGCGGAAATGGCTTACGAACGGGGCGGAGATT
TCCTGGAAGATGCCAGGAAGATACTTAACAGGGAAGTGA
GAGGGCCGCGGCAAAGCCGTTTTTCCATAGGCTCCGCCC
CCCTGACAAGCATCACGAAATCTGACGCTCAAATCAGTG
GTGGCGAAACCCGACAGGACTATAAAGATACCAGGCGTT
TCCCCCTGGCGGCTCCCTCGTGCGCTCTCCTGTTCCTGC
CTTTCGGTTTACCGGTGTCATTCCGCTGTTATGGCCGCG
TTTGTCTCATTCCACGCCTGACACTCAGTTCCGGGTAGG
CAGTTCGCTCCAAGCTGGACTGTATGCACGAACCCCCCG
TTCAGTCCGACCGCTGCGCCTTATCCGGTAACTATCGTC
TTGAGTCCAACCCGGAAAGACATGCAAAAGCACCACTGG
CAGCAGCCACTGGTAATTGATTTAGAGGAGTTAGTCTTG
AAGTCATGCGCCGGTTAAGGCTAAACTGAAAGGACAAGT
TTTGGTGACTGCGCTCCTCCAAGCCAGTTACCTCGGTTC
AAAGAGTTGGTAGCTCAGAGAACCTTCGAAAAACCGCCC
TGCAAGGCGGTTTTTTCGTTTTCAGAGCAAGAGATTACG
CGCAGACCAAAACGATCTCAAGAAGATCATCTTATTAAT
CAGATAAAATATT

| 137 | Backbone. Containing a P15A origin, Kanamycin resistance gene, chloramphenico l resistance gene and mRFP driven by a tac promoter. The backbone sequence contains 2 SapI sites that excise the chloramphenico l resistance gene and leave the overhangs for insertion of the 3' UTR. | TCTAGATTTCAGTGCAATTTATCTCTTCAAATGTAGCACC TGAAGTCAGCCCCATACGATATAAGTTGTAATTCGGTAC CCCGCTTCGGCGGGGTTTTTTCAAGTTCAAATATGTATCC GCTCATGAGACAATGTGTGGGGAGACCACAACGGTTTCC CTCTAGAAATAATTTTGTTTAACTATAAGAAGGAGATATA CATATGGCTTCCTCCGAAGACGTTATCAAAGAGTTCATG CGTTTCAAAGTTCGTATGGAAGGTTCCGTTAACGGTCAC GAGTTCGAAATCGAAGGTGAAGGTGAAGGTCGTCCGTAC GAAGGTACACAGACCGCTAAACTGAAAGTTACCAAAGGT GGCCCGCTGCCGTTCGCTTGGGACATCCTGTCCCCGCAG TTCCAGTACGGTTCCAAAGCTTACGTTAAACACCCGGCT GACATCCCGGACTACCTGAAACTGTCCTTCCCGGAAGGT TTCAAATGGGAACGTGTTATGAACTTCGAAGACGGTGGT GTTGTTACCGTTACCCAGGACTCCTCCCTGCAAGACGGT GAGTTCATCTACAAAGTTAAACTGCGTGGTACCAACTTC CCGTCCGACGGTCCGGTTATGCAGAAAAAACCATGGGT TGGGAAGCTTCCACCGAACGTATGTACCCGGAAGACGGT GCTCTGAAAGGTGAAATCAAAATGCGTCTGAAACTGAAA GACGGTGGTCACTACGACGCTGAAGTTAAAACCACCTAC ATGGCTAAAAAACCGGTTCAGCTGCCGGGTGCTTACAAA ACCGACATCAAACTGGACATCACCTCCCACAACGAAGAC TACACCATCGTTGAACAGTACGAACGTGCTGAAGGTCGT CACTCCACCGGTGCTTAATGAAGAGCGATCGGCACGTAA GAGGTTCCAACTTTCACCATAATGAAATAAGATCACTACC GGGCGTATTTTTTGAGTTATCGAGATTTTCAGGAGCTAA GGAAGCTAAAATGGAGAAAAAATCACTGGATATACCAC CGTTGATATATCCCAATGGCATCGTAAAGAACATTTTGA GGCATTTCAGTCAGTTGCTCAATGTACCTATAACCAGAC CGTTCAGCTGGATATTACGGCCTTTTTAAAGACCGTAAA GAAAAATAAGCACAAGTTTTATCCGGCCTTTATTCACATT CTTGCCCGCCTGATGAATGCTCATCCGGAATTCCGTATG GCAATGAAAGACGGTGAGCTGGTGATATGGGATAGTGTT CACCCTTGTTACACCGTTTTCCATGAGCAAACTGAAACGT TTTCATCGCTCTGGAGTGAATACCACGACGATTTCCGGC AGTTTCTACACATATATTCGCAAGATGTGGCGTGTTACG GTGAAAACCTGGCCTATTTCCCTAAAGGGTTTATTGAGA ATATGTTTTTCGTCTCAGCCAATCCCTGGGTGAGTTTCAC CAGTTTTGATTTAAACGTGGCCAATATGGACAACTTCTTC GCCCCGTTTTCACCATGGGCAAATATTATACGCAAGGC GACAAGGTGCTGATGCCGCTGGCGATTCAGGTTCATCAT GCCGTCTGTGATGGCTTCCATGTCGGCAGAATGCTTAAT GAATTACAACAGTACTGCGATGAGTGGCAGGGCGGGGC GTAATTTTTTTAAGGCAGTTATTGGTGCCCTTAAACGCGC TCTTCTCCCCGCTTCGGCGGGGTTTTTTTGCAAGTGGCA CTTTTCGGGGAAATGTGCGCGGAACCCCTATTTGTTTATT TTTCTAAATACATTCAAATATGTATCCGCTCATGAATTAA TTCTTAGAAAAACTCATCGAGCATCAAATGAAACTGCAAT TTATTCATATCAGGATTATCAATACCATATTTTTGAAAAA GCCGTTTCTGTAATGAAGGAGAAAACTCACCGAGGCAGT TCCATAGGATGGCAAGATCCTGGTATCGGTCTGCGATTC CGACTCGTCCAACATCAATACAACCTATTAATTTCCCCTC GTCAAAAATAAGGTTATCAAGTGAGAAATCACCATGAGT |

GACGACTGAATCCGGTGAGAATGGCAAAAGTTTATGCAT
TTCTTTCCAGACTTGTTCAACAGGCCAGCCATTACGCTCG
TCATCAAAATCACTCGCATCAACCAAACCGTTATTCATTC
GTGATTGCGCCTGAGCGAGACGAAATACGCGGTCGCTGT
TAAAAGGACAATTACAAACAGGAATCGAATGCAACCGGC
GCAGGAACACTGCCAGCGCATCAACAATATTTTCACCTG
AATCAGGATATTCTTCTAATACCTGGAATGCTGTTTTCCC
GGGGATCGCAGTGGTGAGTAACCATGCATCATCAGGAGT
ACGGATAAATGCTTGATGGTCGGAAGAGGCATAAATTC
CGTCAGCCAGTTTAGTCTGACCATCTCATCTGTAACATCA
TTGGCAACGCTACCTTTGCCATGTTTCAGAAACAACTCTG
GCGCATCGGGCTTCCCATACAATCGATAGATTGTCGCAC
CTGATTGCCCGACATTATCGCGAGCCCATTTATACCCATA
TAAATCAGCATCCATGTTGGAATTTAATCGCGGCCTAGA
GCAAGACGTTTCCCGTTGAATATGGCTCATACTCTTCCTT
TTTCAATATTATTGAAGCATTTATCAGGGTTATTGTCTCA
TGAGCGGATACATATTTGAATGTATTTAGAAAAATAAACA
AATAGGCTGTCCCTCCTGTTCAGCTACTGACGGGGTGGT
GCGTAACGGCAAAAGCACCGCCGGACATCAGCGCTAGC
GGAGTGTATACTGGCTTACTATGTTGGCACTGATGAGGG
TGTCAGTGAAGTGCTTCATGTGGCAGGAGAAAAAAGGCT
GCACCGGTGCGTCAGCAGAATATGTGATACAGGATATAT
TCCGCTTCCTCGCTCACTGACTCGCTACGCTCGGTCGTT
CGACTGCGGCGAGCGGAAATGGCTTACGAACGGGGCGG
AGATTTCCTGGAAGATGCCAGGAAGATACTTAACAGGGA
AGTGAGAGGGCCGCGGCAAAGCCGTTTTTCCATAGGCTC
CGCCCCCCTGACAAGCATCACGAAATCTGACGCTCAAAT
CAGTGGTGGCGAAACCCGACAGGACTATAAAGATACCAG
GCGTTTCCCCCTGGCGGCTCCCTCGTGCGCTCTCCTGTT
CCTGCCTTTCGGTTTACCGGTGTCATTCCGCTGTTATGGC
CGCGTTTGTCTCATTCCACGCCTGACACTCAGTTCCGGG
TAGGCAGTTCGCTCCAAGCTGGACTGTATGCACGAACCC
CCCGTTCAGTCCGACCGCTGCGCCTTATCCGGTAACTAT
CGTCTTGAGTCCAACCCGGAAAGACATGCAAAAGCACCA
CTGGCAGCAGCCACTGGTAATTGATTTAGAGGAGTTAGT
CTTGAAGTCATGCGCCGGTTAAGGCTAAACTGAAAGGAC
AAGTTTTGGTGACTGCGCTCCTCCAAGCCAGTTACCTCG
GTTCAAAGAGTTGGTAGCTCAGAGAACCTTCGAAAACC
GCCCTGCAAGGCGGTTTTTTCGTTTTCAGAGCAAGAGAT
TACGCGCAGACCAAAACGATCTCAAGAAGATCATCTTAT
TAATCAGATAAAATATT

| 138 | Backbone. Containing a P15A origin, Kanamycin resistance gene, chloramphenicol resistance gene and LacZ driven by a tac promoter. The backbone sequence contains 2 SapI sites that excise the chloramphenicol resistance gene and leave the overhangs for insertion of the 3' UTR. | TCTAGATTTCAGTGCAATTTATCTCTTCAAATGTAGCACC<br>TGAAGTCAGCCCCATACGATATAAGTTGTAATTCGGTAC<br>CCCGCTTCGGCGGGGTTTTTTCAAGTTCAAATATGTATCC<br>GCTCATGAGACAATGTGTGGGGAGACCACAACGGTTTCC<br>CTCTAGAAATAATTTTGTTTAACTATAAGAAGGAGATATA<br>CATATGACCATGATTACGGATTCACTGGCCGTCGTTTTAC<br>AACGTCGTGACTGGGAAAACCCTGGCGTTACCCAACTTA<br>ATCGCCTTGCAGCACATCCCCCTTTCGCCAGCTGGCGTA<br>ATAGCGAAGAGGCCCGCACCGATCGCCCTTCCCAACAGT<br>TGCGCAGCCTGAATGGCGAATGGCGCTTTGCCTGGTTTC<br>CGGCACCAGAAGCGGTGCCGGAAAGCTGGCTGGAGTGC<br>GATCTTCCTGAGGCCGATACTGTCGTCGTCCCCTCAAAC<br>TGGCAGATGCACGGTTACGATGCGCCCATCTACACCAAC<br>GTGACCTATCCCATTACGGTCAATCCGCCGTTTGTTCCCA<br>CGGAGAATCCGACGGGTTGTTACTCGCTCACATTTAATG<br>TTGATGAAAGCTGGCTACAGGAAGGCCAGACGCGAATTA<br>TTTTTGATGGCGTTAACTCGGCGTTTCATCTGTGGTGCAA<br>CGGGCGCTGGGTCGGTTACGGCCAGGACAGTCGTTTGCC<br>GTCTGAATTTGACCTGAGCGCATTTTTACGCGCCGGAGA<br>AAACCGCCTCGCGGTGATGGTGCTGCGCTGGAGTGACG<br>GCAGTTATCTGGAAGATCAGGATATGTGGCGGATGAGCG<br>GCATTTTCCGTGACGTCTCGTTGCTGCATAAACCGACTA<br>CACAAATCAGCGATTTCCATGTTGCCACTCGCTTTAATGA<br>TGATTTCAGCCGCGCTGTACTGGAGGCTGAAGTTCAGAT<br>GTGCGGCGAGTTGCGTGACTACCTACGGGTAACAGTTTC<br>TTTATGGCAGGGTGAAACGCAGGTCGCCAGCGGCACCGC<br>GCCTTTCGGCGGTGAAATTATCGATGAGCGTGGTGGTTA<br>TGCCGATCGCGTCACACTACGTCTGAACGTCGAAAACCC<br>GAAACTGTGGAGCGCCGAAATCCCGAATCTCTATCGTGC<br>GGTGGTTGAACTGCACACCGCCGACGGCACGCTGATTGA<br>AGCAGAAGCCTGCGATGTCGGTTTCCGCGAGGTGCGGAT<br>TGAAAATGGTCTGCTGCTGCTGAACGGCAAGCCGTTGCT<br>GATTCGAGGCGTTAACCGTCACGAGCATCATCCTCTGCA<br>TGGTCAGGTCATGGATGAGCAGACGATGGTGCAGGATAT<br>CCTGCTGATGAAGCAGAACAACTTTAACGCCGTGCGCTG<br>TTCGCATTATCCGAACCATCCGCTGTGGTACACGCTGTG<br>CGACCGCTACGGCCTGTATGTGGTGGATGAAGCCAATAT<br>TGAAACCCACGGCATGGTGCCAATGAATCGTCTGACCGA<br>TGATCCGCGCTGGCTACCGGCGATGAGCGAACGCGTAAC<br>GCGAATGGTGCAGCGCGATCGTAATCACCCGAGTGTGAT<br>CATCTGGTCGCTGGGGAATGAATCAGGCCACGGCGCTAA<br>TCACGACGCGCTGTATCGCTGGATCAAATCTGTCGATCC<br>TTCCCGCCCGGTGCAGTATGAAGGCGGCGGAGCCGACA<br>CCACGGCCACCGATATTATTTGCCCGATGTACGCGCGCG<br>TGGATGAAGACCAGCCCTTCCCGGCTGTGCCGAAATGGT<br>CCATCAAAAAATGGCTTTCGCTACCTGGAGAGACGCGCC<br>CGCTGATCCTTTGCGAATACGCCCACGCGATGGGTAACA<br>GTCTTGGCGGTTTCGCTAAATACTGGCAGGCGTTTCGTC<br>AGTATCCCCGTTTACAGGGCGGCTTCGTCTGGGACTGGG<br>TGGATCAGTCGCTGATTAAATATGATGAAAACGGCAACC<br>CGTGGTCGGCTTACGGCGGTGATTTTGGCGATACGCCGA<br>ACGATCGCCAGTTCTGTATGAACGGTCTGGTCTTTGCCG |

```
ACCGCACGCCGCATCCAGCGCTGACGGAAGCAAAACACC
AGCAGCAGTTTTTCCAGTTCCGTTTATCCGGGCAAACCA
TCGAAGTGACCAGCGAATACCTGTTCCGTCATAGCGATA
ACGAGCTCCTGCACTGGATGGTGGCGCTGGATGGTAAGC
CGCTGGCAAGCGGTGAAGTGCCTCTGGATGTCGCTCCAC
AAGGTAAACAGTTGATTGAACTGCCTGAACTACCGCAGC
CGGAGAGCGCCGGGCAACTCTGGCTCACAGTACGCGTA
GTGCAACCGAACGCGACCGCATGGTCAGAAGCCGGGCA
CATCAGCGCCTGGCAGCAGTGGCGTCTGGCGGAAAACCT
CAGTGTGACGCTCCCCGCCGCGTCCCACGCCATCCCGCA
TCTGACCACCAGCGAAATGGATTTTTGCATCGAGCTGGG
TAATAAGCGTTGGCAATTTAACCGCCAGTCAGGCTTTCTT
TCACAGATGTGGATTGGCGATAAAAAACAACTGCTGACG
CCGCTGCGCGATCAGTTCACCCGTGCACCGCTGGATAAC
GACATTGGCGTAAGTGAAGCGACCCGCATTGACCCTAAC
GCCTGGGTCGAACGCTGGAAGGCGGCGGGCCATTACCA
GGCCGAAGCAGCGTTGTTGCAGTGCACGGCAGATACACT
TGCTGATGCGGTGCTGATTACGACCGCTCACGCGTGGCA
GCATCAGGGGAAAACCTTATTTATCAGCCGGAAAACCTA
CCGGATTGATGGTAGTGGTCAAATGGCGATTACCGTTGA
TGTTGAAGTGGCGAGCGATACACCGCATCCGGCGCGGAT
TGGCCTGAACTGCCAGCTGGCGCAGGTAGCAGAGCGGG
TAAACTGGCTCGGATTAGGGCCGCAAGAAAACTATCCCG
ACCGCCTTACTGCCGCCTGTTTTGACCGCTGGGATCTGC
CATTGTCAGACATGTATACCCCGTACGTCTTCCCGAGCG
AAAACGGTCTGCGCTGCGGGACGCGCGAATTGAATTATG
GCCCACACCAGTGGCGCGGCGACTTCCAGTTCAACATCA
GCCGCTACAGTCAACAGCAACTGATGGAAACCAGCCATC
GCCATCTGCTGCACGCGGAAGAAGGCACATGGCTGAATA
TCGACGGTTTCCACATGGGGATTGGTGGCGACGACTCCT
GGAGCCCGTCAGTATCGGCGGAATTCCAGCTGAGCGCCG
GTCGCTACCATTACCAGTTGGTCTGGTGTCAAAAATAAT
GAAGAGCGATCGGCACGTAAGAGGTTCCAACTTTCACCA
TAATGAAATAAGATCACTACCGGGCGTATTTTTTGAGTTA
TCGAGATTTTCAGGAGCTAAGGAAGCTAAAATGGAGAAA
AAAATCACTGGATATACCACCGTTGATATATCCCAATGG
CATCGTAAAGAACATTTTGAGGCATTTCAGTCAGTTGCTC
AATGTACCTATAACCAGACCGTTCAGCTGGATATTACGG
CCTTTTTAAAGACCGTAAAGAAAAATAAGCACAAGTTTTA
TCCGGCCTTTATTCACATTCTTGCCCGCCTGATGAATGCT
CATCCGGAATTCCGTATGGCAATGAAAGACGGTGAGCTG
GTGATATGGGATAGTGTTCACCCTTGTTACACCGTTTTCC
ATGAGCAAACTGAAACGTTTTCATCGCTCTGGAGTGAAT
ACCACGACGATTTCCGGCAGTTTCTACACATATATTCGCA
AGATGTGGCGTGTTACGGTGAAAACCTGGCCTATTTCCC
TAAAGGGTTTATTGAGAATATGTTTTTCGTCTCAGCCAAT
CCCTGGGTGAGTTTCACCAGTTTTGATTTAAACGTGGCC
AATATGGACAACTTCTTCGCCCCCGTTTTCACCATGGGC
AAATATTATACGCAAGGCGACAAGGTGCTGATGCCGCTG
GCGATTCAGGTTCATCATGCCGTCTGTGATGGCTTCCAT
GTCGGCAGAATGCTTAATGAATTACAACAGTACTGCGAT
GAGTGGCAGGGCGGGGCGTAATTTTTTTAAGGCAGTTAT
```

| | | TGGTGCCCTTAAACGCGCTCTTCTCCCCGCTTCGGCGGG |
| --- | --- | --- |
| | | GTTTTTTTGCAAGTGGCACTTTTCGGGGAAATGTGCGCG |
| | | GAACCCCTATTTGTTTATTTTTCTAAATACATTCAAATAT |
| | | GTATCCGCTCATGAATTAATTCTTAGAAAAACTCATCGAG |
| | | CATCAAATGAAACTGCAATTTATTCATATCAGGATTATCA |
| | | ATACCATATTTTTGAAAAAGCCGTTTCTGTAATGAAGGAG |
| | | AAAACTCACCGAGGCAGTTCCATAGGATGGCAAGATCCT |
| | | GGTATCGGTCTGCGATTCCGACTCGTCCAACATCAATAC |
| | | AACCTATTAATTTCCCTCGTCAAAAATAAGGTTATCAAG |
| | | TGAGAAATCACCATGAGTGACGACTGAATCCGGTGAGAA |
| | | TGGCAAAAGTTTATGCATTTCTTTCCAGACTTGTTCAACA |
| | | GGCCAGCCATTACGCTCGTCATCAAAATCACTCGCATCA |
| | | ACCAAACCGTTATTCATTCGTGATTGCGCCTGAGCGAGA |
| | | CGAAATACGCGGTCGCTGTTAAAAGGACAATTACAAACA |
| | | GGAATCGAATGCAACCGGCGCAGGAACACTGCCAGCGC |
| | | ATCAACAATATTTTCACCTGAATCAGGATATTCTTCTAAT |
| | | ACCTGGAATGCTGTTTTCCCGGGGATCGCAGTGGTGAGT |
| | | AACCATGCATCATCAGGAGTACGGATAAAATGCTTGATG |
| | | GTCGGAAGAGGCATAAATTCCGTCAGCCAGTTTAGTCTG |
| | | ACCATCTCATCTGTAACATCATTGGCAACGCTACCTTTGC |
| | | CATGTTTCAGAAACAACTCTGGCGCATCGGGCTTCCCAT |
| | | ACAATCGATAGATTGTCGCACCTGATTGCCCGACATTAT |
| | | CGCGAGCCCATTTATACCCATATAAATCAGCATCCATGTT |
| | | GGAATTTAATCGCGGCCTAGAGCAAGACGTTTCCCGTTG |
| | | AATATGGCTCATACTCTTCCTTTTTCAATATTATTGAAGC |
| | | ATTTATCAGGGTTATTGTCTCATGAGCGGATACATATTTG |
| | | AATGTATTTAGAAAAATAAACAAATAGGCTGTCCCTCCTG |
| | | TTCAGCTACTGACGGGGTGGTGCGTAACGGCAAAAGCAC |
| | | CGCCGGACATCAGCGCTAGCGGAGTGTATACTGGCTTAC |
| | | TATGTTGGCACTGATGAGGGTGTCAGTGAAGTGCTTCAT |
| | | GTGGCAGGAGAAAAAAGGCTGCACCGGTGCGTCAGCAG |
| | | AATATGTGATACAGGATATATTCCGCTTCCTCGCTCACTG |
| | | ACTCGCTACGCTCGGTCGTTCGACTGCGGCGAGCGGAAA |
| | | TGGCTTACGAACGGGGCGGAGATTTCCTGGAAGATGCCA |
| | | GGAAGATACTTAACAGGGAAGTGAGAGGGCCGCGGCAA |
| | | AGCCGTTTTTCCATAGGCTCCGCCCCCCTGACAAGCATC |
| | | ACGAAATCTGACGCTCAAATCAGTGGTGGCGAAACCCGA |
| | | CAGGACTATAAAGATACCAGGCGTTTCCCCCTGGCGGCT |
| | | CCCTCGTGCGCTCTCCTGTTCCTGCCTTTCGGTTTACCGG |
| | | TGTCATTCCGCTGTTATGGCCGCGTTTGTCTCATTCCACG |
| | | CCTGACACTCAGTTCCGGGTAGGCAGTTCGCTCCAAGCT |
| | | GGACTGTATGCACGAACCCCCGTTCAGTCCGACCGCTG |
| | | CGCCTTATCCGGTAACTATCGTCTTGAGTCCAACCCGGA |
| | | AAGACATGCAAAAGCACCACTGGCAGCAGCCACTGGTAA |
| | | TTGATTTAGAGGAGTTAGTCTTGAAGTCATGCGCCGGTT |
| | | AAGGCTAAACTGAAAGGACAAGTTTTGGTGACTGCGCTC |
| | | CTCCAAGCCAGTTACCTCGGTTCAAAGAGTTGGTAGCTC |
| | | AGAGAACCTTCGAAAAACCGCCCTGCAAGGCGGTTTTTT |
| | | CGTTTTCAGAGCAAGAGATTACGCGCAGACCAAAACGAT |
| | | CTCAAGAAGATCATCTTATTAATCAGATAAAATATT |

Figure 6

Europäisches
Patentamt

European
Patent Office

Office européen
des brevets

**EUROPEAN SEARCH REPORT**

Application Number

EP 20 20 3857

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/272924 A1 (INOUYE MASAYORI [US] ET AL) 8 December 2005 (2005-12-08)<br>* paragraphs [0063], [0099]; figure 1 * | 1-15 | INV.<br>C12N15/63 |
| X | NIEUWKOOP THIJS ET AL: "The Ongoing Quest to Crack the Genetic Code for Protein Production",<br>MOLECULAR CELL, ELSEVIER, AMSTERDAM , NL,<br>vol. 80, no. 2,<br>2 October 2020 (2020-10-02), pages 193-209, XP086297587,<br>ISSN: 1097-2765, DOI:<br>10.1016/J.MOLCEL.2020.09.014<br>[retrieved on 2020-10-02] | 1,2 | |
| A | * figure 4 * | 3-15 | |
| A | BRIAN F PFLEGER ET AL: "Combinatorial engineering of intergenic regions in operons tunes expression of multiple genes",<br>NATURE BIOTECHNOLOGY,<br>vol. 24, no. 8, 1 August 2006 (2006-08-01), pages 1027-1032, XP055046133,<br>ISSN: 1087-0156, DOI: 10.1038/nbt1226<br>* figure 1 * | 1-15 | |
| | | | TECHNICAL FIELDS SEARCHED (IPC) |
| | | | C12N<br>C40B |

-/--

~~The present search report has been drawn up for all claims~~

| Place of search | Date of completion of the search | Examiner |
|---|---|---|
| Munich | 27 April 2021 | Luo, Xinmei |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

**EUROPEAN SEARCH REPORT**

Application Number

EP 20 20 3857

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE EMBL [Online]<br><br>31 December 2005 (2005-12-31),<br>"46306 Tomato HindIII BAC Library Lycopersicon esculentum genomic clone LE_HBa0058M18 3, genomic survey sequence.",<br>XP002802623,<br>retrieved from EBI accession no. EM_GSS:DU851122<br>Database accession no. DU851122<br>* The query sequence SEQ ID NO:2 has 100.00 % identity (100.00 % similarity) over 20 positions in a common overlap (range (q:s): 1-20:651-670) with subject EM_GSS:DU851122 (length: 878).; sequence * | 1-15 | |
| | ----- | | |
| A | WO 2013/142033 A1 (METABOLIX INC [US]) 26 September 2013 (2013-09-26)<br>* paragraph [0108] - paragraph [0112] *<br>----- | 1-15 | TECHNICAL FIELDS SEARCHED (IPC) |

~~The present search report has been drawn up for all claims~~

| Place of search | Date of completion of the search | Examiner |
|---|---|---|
| Munich | 27 April 2021 | Luo, Xinmei |

Europäisches
Patentamt

European
Patent Office

Office européen
des brevets

Application Number

EP 20 20 3857

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 20 20 3857

The Search Division considers that the present European patent application does not comply with the
requirements of unity of invention and relates to several inventions or groups of inventions, namely:

1. claims: 1-15(partially)

    relates to a prokaryotic expression cassette, comprising an
    expression cassette for expression of a protein of interest,
    said expression cassette comprising a promoter, a
    poly-linker, a 3' untranslated region (UTR) comprising a
    nucleotide sequence according to SEQ ID NO:2, and a
    terminator;
    ---

2-56. claims: 1-15(partially)

    as invention 1, wherein the 3' untranslated region (UTR)
    comprising a nucleotide sequence according to SEQ ID
    NOs:3-57, respectively.
    ---

**ANNEX TO THE EUROPEAN SEARCH REPORT
ON EUROPEAN PATENT APPLICATION NO.**

EP 20 20 3857

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-2021

| Patent document cited in search report | Publication date | Patent family member(s) | Publication date |
|---|---|---|---|
| US 2005272924 A1 | 08-12-2005 | AU 2003216384 A1 | 16-09-2003 |
| | | CA 2477856 A1 | 12-09-2003 |
| | | CN 1639320 A | 13-07-2005 |
| | | EP 1478733 A2 | 24-11-2004 |
| | | JP 4249031 B2 | 02-04-2009 |
| | | JP 2005518804 A | 30-06-2005 |
| | | KR 20040086424 A | 08-10-2004 |
| | | TW 200401029 A | 16-01-2004 |
| | | US 2005272924 A1 | 08-12-2005 |
| | | WO 03074657 A2 | 12-09-2003 |
| WO 2013142033 A1 | 26-09-2013 | BR 112014023317 A2 | 18-07-2017 |
| | | CN 104321427 A | 28-01-2015 |
| | | EP 2828383 A1 | 28-01-2015 |
| | | US 2015159184 A1 | 11-06-2015 |
| | | WO 2013142033 A1 | 26-09-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2001027293 A


**Non-patent literature cited in the description**

- **GALPERIN ; KOONIN.** *Nat Biotechnol,* 2000, vol. 10, 1038, , 76443
- **HUYNEN et al.** *Genome Res 10.1101/gr.10.8.1204,* 2000
- **OPPENHEIM ; YANOFSKY.** *Genetics,* 1980, vol. 95, 785-795
- **SCHÜMPERLI et al.** *Cell,* 1982, vol. 30, 865-871
- **AKSOY et al.** *J Bacteriol,* 1984, vol. 157, 363-367
- **LEVIN-KARP et al.** *ACS Synth Biol,* 2013, vol. 2, 327-336
- **GOVANTES et al.** *EMBO J,* 1998, vol. 17, 2368-2377
- **REX et al.** *J Biol Chem,* 1994, vol. 269, 18118-18127
- **QUAX et al.** *Cell Rep 10.1016/j.celrep.2013.07.04924,* 2013
- **SALIS.** *Methods Enzymol,* 2011, vol. 498, 19-42
- **LI et al.** *Nucleic. Acids Res.,* 2016, vol. 44, 2554-2563
- **CRAMERI et al.** *Nat Biotechnol,* 1996, vol. 14, 315-9
- **CAMPBELL et al.** *PNAS,* 2002, vol. 99, 7877-7882
- **DONOHUE et al.** *Trends Biotech,* 2018, vol. 2, 134-146
- **CHEN ; CLOWES.** *Nucl Acids Res.,* 1984, vol. 12, 3219-3234
- **BROSIUS.** *Gene,* 1984, vol. 27, 151-160
- **KIM et al.** *Biotechnol Bioprocess Engineering,* 2008, vol. 13, 313
- **SØRVIG et al.** *Microbiol,* 2005, vol. 151, 2439-2449
- **DE AVILA E SILVA et al.** *J Theor Biol,* 2011, vol. 287, 92-99
- **DE JONG et al.** *BMC Genomics,* 2012, vol. 13, 299
- **GUIZIOU S. et al.** *Nucl Acids Res,* 2016, vol. 44, 7495-7508
- **ANGOVET.** Heterologous Gene Expression in E.coli. Methods in Molecular Biology (Methods and Protocols). Humana Press, 2011, vol. 705
- **CLAASSENS et al.** *PLoS One,* 2017, vol. 12, e0184355
- The Encyclopedia of Molecular Biology. Blackwell Science Ltd, 1994
- **BENJAMIN LEWIN.** Genes X. Jones & Bartlett Publishing, 2009
- Molecular Biology and Biotechnology: a Comprehensive Desk Reference. VCH Publishers, Inc, 1995
- **COLIGAN et al.** Current Protocols in Protein Sciences. Wiley Intersciences, 2009
- **SAMBROOK et al.** Molecular Cloning: A Laboratory Manual. Cold Spring Harbor Laboratory Press, 2014
- **DAVIS et al.** Basic Methods in Molecular Biology. Elsevier Science Publishing, Inc, 1995
- Methods in Enzymology: Guide to Molecular Cloning Techniques. Academic Press Inc, 1987, vol. 152
- **CARUTHERS.** *Science,* 1985, vol. 230, 281-285
- **ENGHIAD ; ZHAO.** *ACS Synth Biol,* 2017, vol. 6, 752-757
- **VINCENT et al.** *EMBO Rep,* 2004, vol. 5, 795-800
- **NOTOMI et al.** *Nucleic Acids Res,* 2000, vol. 28, E63
- **GUATELLI et al.** *Proc Natl Acad Sci USA,* 1990, vol. 87, 1874-1878
- **ALI et al.** *Chem Soc Rev,* 2014, vol. 43, 3324-3341
- **WALKER et al.** *Nucleic Acids Res,* 1992, vol. 20, 1691-6
- **PIEPENBURG et al.** *PLoS Biology,* 2006, vol. 4, e204
- **CRAMERI et al.** *Nat. Biotechnol,* 1996, vol. 14, 315-9
- **LORENZ ; WACKERNAGEL.** *Microbiol Rev,* 1994, vol. 58, 563-602
- **WIRTH et al.** *Mol Gen Genetics,* 1989, vol. 216, 175-7
- Collins and Lyne's Microbiological Methods. Arnold, 2004
- **COULSON.** *J Mol Biol,* 1975, vol. 94, 441-8

- **MAXAM ; GILBERT.** *PNAS USA,* 1977, vol. 74, 560-64
- **NYREN ; UHLEN.** *Anal Biochem,* 1993, vol. 208, 171-175
- **LEVENE et al.** *Science,* 2003, vol. 299, 682-6
- **PENNISI.** *Science,* 2012, vol. 336, 534-537
- Bacterial Secretion Systems: An Overview. ASM Science. 213-239
- **BRINKROLF et al.** *J. Biotech,* 2010, vol. 149, 173-82
- Cytokine and Protein User's Guide Book. R&D systems. 2018
- **FERRER-MIRALLES ; VILLAVERDE.** *Microb Cell Fact,* 2013, vol. 12, 113
- **BALTACI et al.** *Geomicrobiol J,* 2017, vol. 34, 53-62
- **STAALS et al.** *Mol. Cell,* 2013, vol. 52, 135-145
- **STAALS et al.** *Mol. Cell,* 2014, vol. 56, 518-530
- **PERALTA-YAHYA ; KEASLING.** *Biotech J,* 2010, vol. 5, 147-162
- **SADHU ; TUSHAR.** Cellulase production by bacteria: a review. *Microbiol Res J Int,* 2013, vol. 3, 235-258
- **PAULA et al.** *J. of King Saud Univ-Science,* 2017, vol. 29, 166-173
- **EWING et al.** *Genome Res,* 1998, vol. 8, 175-185
- **ZHANG ; BREMER.** *J Biol Chem,* 1995, vol. 270, 11181-11189